# DEMANDE DE BREVET EUROPEEN

(11) **EP 3 689 384 A1**
(43) Date de publication de la demande: **05.08.2020**
(21) Numéro de dépôt: 20156278.2
(22) Date de dépôt: 12.04.2013
(51) Int. Cl.: A61K 49/00, A61K 49/10, A61K 49/18, A61K 51/12

(54) **NANOPARTICULES ULTRAFINES COMME AGENT DE CONTRASTE MULTIMODAL**

(30) Priorité: 13.04.2012 FR 1253438
(62) Demande divisionnaire de: 13718146.7
(71) Demandeur: CENTRE NATIONAL DE LA RECHERCHE SCIENTIFIQUE - CNRS -, 75794 Paris Cedex 16 (FR); Université Claude Bernard Lyon 1, 69622 Villeurbanne Cedex (FR); NANOH, 38070 Saint Quentin Fallavier (FR); Université Grenoble Alpes, 38400 Saint-Martin-d'Hères (FR); Université de Bordeaux, 33000 Bordeaux (FR)
(72) Inventeur: CREMILLIEUX, Yannick, 33200 Bordeaux (FR); BIANCHI, Andrea, 88400 Biberach an der Riss (DE); DUFORT, Sandrine, 38100 Grenoble (FR); COLL, Jean-Luc, 38700 La Tronche (FR); LUX, François, 69003 Lyon (FR); TILLEMENT, Olivier, 69270 Fontaines Saint-Martin (FR)
(74) Mandataire: Plasseraud IP

(57) **Abrégé**

L'invention concerne une nouvelle utilisation de nanoparticules ultrafines, utiles comme agent diagnostic, thérapeutique ou théranostic, caractérisée par leur mode d'administration par les voies aériennes. L'invention vise également les applications découlant de ce nouveau mode d'administration, notamment pour l'imagerie des poumons, et le diagnostic ou pronostic des pathologies pulmonaires. Dans le domaine thérapeutique, les applications envisagées sont celles d'agents radio-sensibilisants ou radioactifs pour la radiothérapie (et éventuellement la curiethérapie), pour la neutronthérapie, d'agents pour la PDT (photodynamic therapy), notamment pour le traitement des tumeurs du poumon.

## Description

### Domaine technique

L'invention concerne une nouvelle utilisation de nanoparticules ultrafines, comme agent diagnostic, thérapeutique ou théranostic, caractérisée par leur mode d'administration par les voies aériennes. L'invention vise également les applications découlant de ce nouveau mode d'administration, notamment pour l'imagerie des poumons, et le diagnostic ou pronostic des pathologies pulmonaires. Dans le domaine thérapeutique, les applications envisagées sont celles d'agents radio-sensibilisants ou radioactifs pour la radiothérapie (et éventuellement la curiethérapie), pour la neutronthérapie, d'agents pour la photothérapie ou thermothérapie, notamment pour le traitement des tumeurs du poumon.

### Arrière-plan technologique

En dépit d'efforts de recherche considérables, le cancer reste l'une des causes majeures de décès dans le monde (R. Siegel et al.: Cancer Statistics 2012, 2012, 62, 10-29). Parmi les différents types de cancer, le cancer du poumon constitue la cause principale de mort associée à un cancer (J. S. Guthi et al., Molecular Pharmaceutics, 2009, 7, 32-40) :
- on compte 1.4 millions de décès dans le monde chaque année,
- le taux de survie à 5 ans est inférieur à 15%.

La faible survie des patients atteints du cancer du poumon est principalement due à l'absence d'outils pour le diagnostic précoce et pour cibler localement les agents thérapeutiques.

L'utilisation de nanoparticules comme agent de contraste en imagerie médicale ou agent thérapeutique est connue depuis plus de deux décennies. Ces nanoparticules présentent en effet de nombreux avantages par rapport aux composés moléculaires :
elles permettent une approche multimodale (M. Lewin et al., Nat. Biotechnol., 2000, 18, 410-414),
- la détection est nettement améliorée en raison du plus grand nombre d'éléments actifs par particules, de plus dans le cadre de l'IRM, l'efficacité par ion Gd³⁺ est également améliorée (P. Caravan, Chem. Soc. Rev., 2006, 35, 512-523 / J. S. Ananta et al., Nat. Nano., 2010, 5, 815-821),
- on peut greffer plusieurs molécules d'un ligand particulier par nanoparticule et/ou combiner plusieurs types de ligands par nanoparticules pour augmenter l'affinité des nanoparticules pour certains tissus ou types cellulaires en fonction de l'application envisagée (E. Garanger et al., Org. Biomol. Chem., 2006, 4, 1958-1964 / Z.-H. Jin et al., Molecular Cancer, 2007, 6, 41),
- leur échelle nanométrique leur confèrent des propriétés nouvelles et originales utilisables pour les applications biomédicales (E. Boisselier, D. Astruc, Chem. Soc. Rev., 2009, 38, 1759-1782 / C. Xu, S. Sun, Dalton Trans., 2009, 5583-5591 /P. Zrazhevskiy et al., Chem. Soc. Rev., 2010, 39, 4326-4354*),*
- elles sont rigides, et présentent peu d'interaction avec le milieu biologique.

L'approche multimodale consiste en particulier à mettre en œuvre un ensemble de nanoparticules comprenant, chacune, plusieurs agents de contraste moléculaires. Elle permet ainsi de combiner non seulement différentes techniques d'imagerie, mais aussi différentes techniques de thérapie en regroupant plusieurs agents actifs en thérapie dans une même nanoparticule. Les agents actifs en imagerie et les agents actifs en thérapie peuvent être identiques ou différents entre eux.

Cette approche est particulièrement adaptée pour le développement de médicaments en théranostic. On peut notamment ajouter d'autres fonctions d'imagerie (luminescence, scintigraphie...), des fonctions thérapeutiques (largage de principes actifs, radio-sensibilisation, curie-thérapie...) ainsi que des fonctions de ciblage biologique pour une concentration des agents thérapeutiques dans la zone d'intérêt. Cette approche permet notamment d'envisager une thérapie guidée par l'imagerie en déterminant précisément le comportement de l'agent théranostic dans le corps grâce à sa biodistribution visualisée par imagerie. L'agent theranostic peut ensuite être activé (par les rayons X, y, des neutrons, la lumière suivant le type d'agent) au meilleur moment (lorsque la concentration est maximale dans la zone à traiter et minimale dans les tissus sains).

La demande de brevet WO 2009/053644 décrit des nanoparticules à base de lanthanides (gadolinium en particulier) et leurs utilisations comme agents radio-sensibilisants. Elle divulgue l'utilisation de molécules organiques à la surface ou dans l'enrobage des nanoparticules, de sorte notamment à améliorer la biodistribution et favoriser les surconcentrations locales dans les zones tumorales.

La demande de brevet WO 2011/135102 décrit des nanoparticules ultrafines, d'un diamètre moyen inférieur à 5 nm, à matrice polyorganosiloxane fonctionnalisée et incluant des complexes métalliques, par exemple de gadolinium, et le cas échéant d'autres agents de contraste ou radiosensibilisant. Ces nanoparticules ultrafines présentent des propriétés multimodales particulièrement intéressantes en imagerie médicale et dans la thérapie du cancer. Après une injection en intraveineuse, on constate une excellente biodistribution et une élimination rénale rapide et complète en raison de leur très faible taille, limitant par là les risques d'effets secondaires ou toxiques (F. Lux et al., Ange. Chem. Int. Ed., 2011, 123, 12507-12511).

Le poumon est un organe unique en ce sens qu'il peut être ciblé par un agent diagnostic ou thérapeutique soit via la voie intraveineuse, soit via les voies aériennes. Cependant, l'administration des nanoparticules utilisables comme agent de contraste se fait classiquement par injection intraveineuse en raison des risques de rétention à long terme dans les poumons (et donc d'une toxicité associée). (J. Roller et al., Nanomedicine, 2011, 7, 753-762 /R. Rossin et al., J. Nucl. Med., 2008, 49, 103-111).

En tomographie CT (computed tomography), on a décrit l'administration par inhalation de nanoparticules à base d'éléments lourds (par exemple, l'or (Cai, S. H. et al, Investigative Radiology, 2007, 42, 797-806) et les composés iodés (Aillon, et al, Molecular Pharmaceutics, 2010, 7, 1274-1282*).* En imagerie par résonance magnétique (IRM), seulement des agents de contraste négatifs (nanoparticules à base d'oxydes de fer (G. Huang et al., Chem., 2009, 19, 6367-6372*))* ont été utilisés par voie aérienne du fait de leur faible toxicité et leur facilité de synthèse et de fonctionnalisation. Cependant, l'utilisation d'agent de contraste négatif n'est pas optimale pour l'imagerie du poumon et le diagnostic pourrait être amélioré par l'utilisation d'agents de contraste positif.

Pour l'imagerie en fluorescence, des nanoparticules à émission proche de l'infra-rouge (NIR), telles que les quantum dots ont été utilisées, mais leur toxicité inhérente limite les futures applications cliniques possibles (A. Zintcheko et al., Molecular Therapy, 2009, 17, 1849-1856 / F-X. Blé et al. Magnetic Resonance in Medicine 62:1164-1174 2009) décrivent la synthèse et le comportement *in vivo* de molécules capables de marquer le mucus afin d'étudier les mécanismes d'épuration mucociliaires. Ils montrent l'avantage d'utiliser un fluorophore dans le proche IR ainsi qu'un agent de contraste T₁ pour leurs macromolécules. Cette approche ne permet pas d'obtenir un fort signal IRM (4 Gd³⁺ pour des molécules de plus de 10 kDa) ou alors il est nécessaire d'utiliser des polymères de dextran de masse molaire beaucoup plus élevées (supérieures à 70 kDa). De plus ces polymères de type polysaccharide sont actifs du point de vue biologique et peuvent entrer en interaction avec les glycoprotéines du mucus pour la fixation sur les sites riches en liaisons hydrogènes. L'agent de contraste est éliminé par expectoration et ne permet pas d'obtenir un rehaussement des tissus pulmonaires.

Ainsi, à la connaissance des inventeurs, l'administration par les voies aériennes, d'agents de contraste multimodaux nanoparticulaires à base de gadolinium n'a jamais été décrite pour l'imagerie IRM T₁ des poumons. Les inventeurs ont maintenant montré qu'une administration par les voies aériennes d'agent de contraste sous la forme de nanoparticules ultrafines (d'un diamètre moyen inférieur à 10nm, voire inférieur à 5nm, par exemple compris entre 1 et 5nm) permet une répartition de l'agent de contraste particulièrement favorable pour l'imagerie du poumon ou la thérapie des pathologies pulmonaires et une élimination rénale satisfaisante, limitant ainsi les risques de toxicité, inhérents à ce type de structures.

### Objectifs et brève description de l'invention

La présente invention vise à satisfaire au moins l'un des objectifs suivants :
(i) proposer de nouveaux mode d'administration topique d'agents de contraste multimodaux, en imagerie (e.g. IRM T₁, scintigraphie PET ou SPECT, fluorescence proche IR, tomographie de rayons X), et qui soient faiblement toxiques ;
(ii) proposer de nouvelles méthodes d'imagerie permettant une détection fiable des pathologies pulmonaires, notamment les tumeurs du poumon ou le remodelage bronchique associé à l'asthme sévère, éventuellement couplées à un acte thérapeutique,
(iii) proposer de nouveaux agents diagnostics, thérapeutiques ou théranostics pour les pathologies pulmonaires, et en particulier le cancer du poumon ou l'asthme, lesquels agents présentent un fort potentiel en IRM T₁ conjugué à d'autres modalités d'imagerie, une répartition pulmonaire adaptée, un passage dans le sang et une élimination rénale efficace,
(iv) proposer de nouveaux moyens d'administration de pro-drogue ou d'agent de contraste, nécessitant peu de dispositifs et permettant de réduire les doses à injecter par une voie non-invasive, confortable et non-toxique pour le patient.
(v) proposer de nouveaux moyens de ciblage des tumeurs non pulmonaires, via une administration non-invasive, grâce au passage dans le sang.

Ces objectifs, parmi d'autres, sont atteints par la présente invention qui concerne dans un premier aspect, des nanoparticules pour leur utilisation comme agent thérapeutique ou comme agent de contraste pour l'imagerie médicale,
- d'un diamètre moyen compris entre 1 et 20 nm, de préférence entre 1 et 10 nm, et encore plus préféré entre 2 et 5 nm ou encore entre 1 et 5nm,
- comprenant au moins un agent de contraste pour l'imagerie médicale et/ou un agent radio-sensibilisant ou radioactif pour la radiothérapie, un agent pour la neutronthérapie, la photothérapie; ou, la thermothérapie,
et caractérisées en ce qu'elles sont administrées chez l'homme ou l'animal par les voies aériennes, notamment intranasale ou intratrachéale.

Dans un mode de réalisation préféré, on utilise des nanoparticules hybrides comprenant :
- une matrice de polyorganosiloxane (POS) incluant, à titre d'agent de contraste ou radio-sensibilisant, des cations de terre rare Mⁿ⁺, n étant un nombre entier compris entre 2 et 4, éventuellement en partie sous forme d'un oxyde et/ou d'un oxohydroxyde métallique, éventuellement associé à des cations dopants D^{m+}, m étant un nombre entier compris entre 2 et 6, D étant de préférence un élément de transition ou une terre rare ;
- un agent chélatant greffé à la matrice POS par liaison covalente -Si-C-, en quantité suffisante pour pouvoir complexer tous les cations Mⁿ⁺ et, le cas échéant, D^{m+}; l'agent chélatant ainsi greffé étant de préférence en excès par rapport aux cations Mⁿ⁺ et, le cas échéant, D^{m+};
- le cas échéant, une ou plusieurs molécules ciblantes, pour le ciblage des nanoparticules, lesdites molécules ciblantes étant greffée(s) à la matrice POS ou à l'agent chélatant.

Dans un second aspect, l'invention vise une méthode d'administration non-invasive de nanoparticules hybrides telles que définies ci-dessus, caractérisées en ce que les nanoparticules hybrides sont administrées chez l'homme ou l'animal par les voies aériennes sous forme d'aérosol.

Dans un troisième aspect, l'invention vise un aérosol pour l'administration d'un agent de contraste ou thérapeutique par les voies aériennes, contenant, des nanoparticules telles que définies ci-dessus, et le cas échéant, un véhicule, fluide ou solvant pharmaceutiquement acceptable. L'invention concerne les formulations pharmaceutiques appropriées pour ces aérosols et un dispositif d'aérosolisation les contenant.

Dans un quatrième aspect, l'invention vise une méthode de suivi de l'efficacité thérapeutique d'un traitement thérapeutique chez l'homme ou l'animal, ladite méthode comprenant les étapes suivantes :
(i) à l'initiation du traitement, on administre au patient des nanoparticules telles que définies ci-dessus, à titre d'agent de contraste, par les voies aérienne sous la forme d'aérosol,
(ii) on capture les images par une technique d'imagerie appropriée afin de visualiser les lésions,
(iii)on répète les étapes (i) et (ii) au cours du traitement du patient, autant que nécessaire,
(iv)on déduit l'efficacité thérapeutique du traitement en comparant l'évolution des lésions sur les images au cours du traitement.

Cette méthode s'applique avantageusement au suivi de l'efficacité d'un traitement anti-tumoral, notamment dirigé contre les tumeurs du poumon, ou encore au suivi de l'efficacité d'un traitement contre une affection pulmonaire inflammatoire, telle que l'asthme.

Dans un cinquième aspect, l'invention vise des nanoparticules pour leurs utilisations thérapeutiques contre le cancer du poumon, comme agent radio-sensibilisant, photosensibilisant, radioactifs, en radiothérapie, neutronthérapie, curiethérapie, photothérapie dynamique ou thermothérapie.

### Description détaillée de l'invention

### Nanoparticules mises en œuvre dans les applications selon l'invention

La présente invention découle des avantages surprenants mis en évidence par les inventeurs d'une administration par les voies aériennes de certaines nanoparticules, utilisables comme agent de contraste, notamment en IRM T₁. L'ensemble des applications qui en découlent sont ainsi liées aux caractéristiques chimiques et structurales de ces nanoparticules qui sont décrites ci-après :
L'invention concerne en effet, des nanoparticules, pour leur utilisation comme agent thérapeutique ou comme agent de contraste pour l'imagerie médicale, lesdites nanoparticules présentant un diamètre moyen compris entre 1 et 20 nm, de préférence entre 1 et 10 nm, et encore plus préféré entre 2 et 5 nm ou encore entre 1 et 5nm, et comprenant au moins un agent de contraste pour l'imagerie médicale et/ou un agent radio-sensibilisant ou radioactif pour la radiothérapie, un agent pour la neutronthérapie, la photothérapie, ou, la thermothérapie, lesdites nanoparticules étant caractérisées en ce qu'elles sont administrées chez l'homme ou l'animal par les voies aériennes, notamment intranasale ou intratrachéale.

Selon l'invention, on utilisera avantageusement des nanoparticules de très faible diamètre. On sélectionnera des nanoparticules d'un diamètre moyen par exemple compris entre 1 et 10 nm, et encore plus préféré entre 2 et 5 nm ou entre 1 et 5nm, permettant une excellente répartition de ces nanoparticules dans les poumons (et donc une détection fiable des pathologies pulmonaires), un passage dans le sang et une élimination rénale rapide (et donc une faible toxicité). Dans un mode de réalisation spécifique, lesdites nanoparticules de très faible diamètre, par exemple compris entre 1 et 5nm sont des nanoparticules comprenant au moins une matrice de polyorganosiloxane.

La distribution de taille des nanoparticules est par exemple mesurée à l'aide d'un granulomètre commercial, tel qu'un granulomètre Malvern Zêta sizer Nano-S basé sur la PCS (Photon Corrélation Spectroscopy). Cette distribution est caractérisée par un diamètre hydrodynamique moyen.

Au sens de l'invention, par « diamètre moyen » on entend la moyenne harmonique des diamètres des particules. Une méthode de mesure de ce paramètre est également décrite dans la norme ISO 13321:1996.

Au sens de l'invention, on entend par « agent de contraste » tout produit ou composition utilisé en imagerie médicale dans le but d'augmenter artificiellement le contraste permettant de visualiser une structure anatomique particulière (par exemple certains tissus ou un organe) ou pathologiques (par exemple des tumeurs) des structures voisines ou non-pathologiques. Le principe de fonctionnement de l'agent de contraste dépend de la technique d'imagerie utilisée.

La structure exacte de l'agent de contraste ou de l'agent radio-sensibilisant sera déterminée en fonction de l'application souhaitée.

Dans une variante préférée, pour les applications en imagerie par résonnance magnétique (IRM), les nanoparticules contiennent de préférence des cations métalliques de terre rare, et de manière encore plus préféré, un lanthanide, présentant un comportement magnétique tel que Gd ou Dy (ou Eu pour la CEST), et représentant au moins 10% de l'ensemble des cations métalliques présents dans la particule. Les nanoparticules à base de lanthanide, par exemple, gadolinium, permettent une utilisation comme agent de contraste positif IRM T₁, particulièrement appropriée pour l'imagerie des poumons.

Ainsi, dans un mode de réalisation préféré, les nanoparticules comprennent chacune, un agent de contraste ou radiosensibilisant, choisi parmi un lanthanide, un oxyde et/ou un oxohydroxyde de lanthanide, le lanthanide étant de préférence choisi parmi Dy, Lu, Gd, Ho, Eu, Tb, Nd, Er, Yb ou leurs mélanges, et plus préférentiellement encore le gadolinium.

Avantageusement, et comme cela est bien connu, on pourra combiner l'utilisation des nanoparticules en thérapie et pour une détection in vivo en IRM, permettant par exemple, un monitorage d'une thérapie. De préférence, on choisira à titre d'agent radiosensibilisant uniquement des lanthanides, et incluant, au moins 50% en masse, de gadolinium (Gd), dysprosium (Dy), de lutetium (Lu) ou d'holmium (Ho) ou leurs mélanges, par exemple au moins 50% en masse de gadolinium.

Selon une variante, on utilisera des nanoparticules dans lesquelles, la partie contenant des lanthanides contient à sa périphérie des lanthanides provoquant un signal IRM, par exemple du gadolinium, et au moins un autre lanthanide dans sa partie centrale. Les lanthanides de fort numéro atomique absorbant les rayonnements étant alors préférentiellement situés au centre du cœur de la nanoparticule.

Selon une autre variante, il est connu qu'un certain nombre de lanthanides présente des sections efficaces de capture de neutrons couplée avec une réaction fortement énergétique permettant leur utilisation dans des traitements par neutron-thérapie par exemple contre le cancer. On pourra choisir, si besoin est, dans la thérapie envisagée, un lanthanide présentant une section efficace de capture suffisante à cet effet, de façon à permettre également un traitement par neutron-thérapie. A cet effet, le choix du gadolinium (¹⁵⁷Gd) s'avère particulièrement intéressant et on utilisera des lanthanides comme agent de contraste constitués d'au moins 50% en masse de gadolinium.

Dans un mode de réalisation préféré, les nanoparticules utilisables selon l'invention sont caractérisées en ce qu'elles comprennent au moins un agent de contraste pour l'imagerie IRM T₁, et au moins un autre agent de contraste approprié pour l'une des techniques d'imageries suivantes :
(i) la scintigraphie PET ou SPECT,
(ii) la fluorescence dans le visible ou dans le proche infra-rouge,
(iii) la tomodensitométrie aux rayons X.

Plus préférentiellement encore, on choisit les nanoparticules de sorte qu'elles ont une relaxivité r1 par particule comprise entre 50 et 5000mM-1.s-1 et/ou un rapport massique en Gd d'au moins 5%, par exemple entre 5 et 50%.

Pour l'imagerie en scintigraphie, les nanoparticules comprennent un isotope radioactif susceptible d'être utilisé en scintigraphie, et de préférence choisi dans le groupe constitué par des isotopes radioactifs de In, Tc, Ga, Cu, Zr, Y ou Lu, par exemple : ¹¹¹In, ^{99m}Tc, ⁶⁸Ga, ⁶⁴Cu, ⁸⁹Zr, ⁹⁰Y, ¹⁷⁷Lu.

Pour la fluorescence dans le proche infra-rouge, on pourra utiliser par exemple un lanthanide choisi parmi Nd, Yb ou Er.

Pour la fluorescence dans le visible on peut utiliser un lanthanide choisi parmi Eu ou Tb. Ces agents de contraste ou thérapeutiques sont éventuellement protégés par un enrobage de nature organique, par exemple une matrice de polyorganosiloxane.

Un mode de réalisation porte ainsi sur les nanoparticules hybrides de type cœur-coquille. On connait notamment des nanoparticules de type cœur coquille, à base d'un cœur constitué d'un oxyde de terres rares et d'une matrice de polyorganosiloxanes, éventuellement fonctionnalisée (voir notamment WO 2005/088314, WO 2009/053644).

Les nanoparticules peuvent être fonctionnalisées à l'aide de molécules permettant un ciblage des nanoparticules vers des tissus spécifiques. La nanoparticule peut servir également de vecteur de l'agent radio-sensibilisant et/ou de l'agent de contraste, en fonction des applications visées. Ceux-ci peuvent être couplés à la nanoparticule par des couplages covalents, ou piégés par liaison non-covalente, par exemple par encapsulation ou interaction hydrophile/hydrophobes ou à l'aide d'un agent chélatant.

Dans un mode de réalisation préféré, on utilise des nanoparticules hybrides comprenant :
- une matrice de polyorganosiloxane (POS) incluant, à titre d'agent de contraste ou radio-sensibilisant, des cations de terre rare Mⁿ⁺, n étant un nombre entier compris entre 2 et 4, éventuellement en partie sous forme d'un oxyde et/ou d'un oxohydroxyde métallique, éventuellement associé à des cations dopants D^{m+}, m étant un nombre entier compris entre 2 et 6, D étant de préférence une terre rare différente de M, un actinide et/ou un élément de transition ;
- un agent chélatant greffé à la matrice POS par liaison covalente -Si-C-, en quantité suffisante pour pouvoir complexer tous les cations Mⁿ⁺ et, le cas échéant, D^{m+}; l'agent chélatant ainsi greffé étant de préférence en excès par rapport aux cations Mⁿ⁺ et, le cas échéant, D^{m+};
- le cas échéant, une molécule ciblante, pour le ciblage des nanoparticules, greffée à la matrice POS ou à l'agent chélatant.

Dans le cas d'une structure de type cœur-coquille, la matrice POS forme la couche superficielle entourant le cœur à base de cations métalliques. Son épaisseur peut alors aller de 0,5 à 10 nm, et représenter de 25 à 75% du volume total.

La matrice POS joue un rôle de protection du cœur vis-à-vis du milieu extérieur (notamment contre l'hydrolyse) et elle optimise les propriétés des agents de contrastes (luminescence par exemple). Elle permet également la fonctionnalisation de la nanoparticule, par le greffage des agents chélatants et des molécules ciblantes.

Avantageusement, l'agent chélatant est choisi parmi les produits suivants :
∘ les produits du groupe des acides polycarboxyliques polyaminés et leurs dérivés, et, plus préférentiellement encore dans le sous-groupe comprenant: DOTA, DTPA, EDTA, EGTA, BAPTA, NOTA et leurs mélanges ;
∘ les produits du groupe comprenant porphyrine, chlorine, 1,10-phénanthroline, bipyridine, terpyridine, cyclam, triazacyclononane, leurs dérivés et leurs mélanges;
∘ et leurs mélanges.

Si M est un lanthanide, l'agent chélatant est avantageusement sélectionné parmi ceux qui présentent des propriétés complexantes des lanthanides, en particulier ceux dont la constante de complexation log(K_{C1}) est supérieure à 15, préférentiellement 20. Comme exemples préférés de chélatants, complexant des lanthanides, on peut citer ceux comprenant un motif d'acide diéthylène triamine penta acétique (DTPA), d'acide 1,4,7,10-tetraazacyclododecane-1,4,7,10-tetra acétique (DOTA), d'acide 1,4,7-triazacyclononane-1,4,7 triacétique (NOTA) ou leurs dérivés.

En outre, en fonction de l'application visée, les nanoparticules sont éventuellement dopées par un autre cation métallique de terres rares ou d'actinides, par exemple un lanthanide, voire deux lanthanides différents, l'un au moins étant choisi parmi Eu et Tb.

Parmi les actinides, dans une variante appropriée pour des applications en thérapie liée à des réactions nucléaires, on choisira par exemple un radionucléide parmi Ac, Th, Pa, Np, U et Pu.

De préférence, M et D sont choisis dans les groupes d'éléments suivants : lanthanides, éléments de transition, actinides, éléments des colonnes Ib, IIa, IIIa , IIIb, Va, VIb, VIIb, VIII de la classification périodique selon "The Merck Index - Eleventh édition" ;
de préférence dans les sous-groupes comprenant :
∘ les lanthanides suivants : Gd, Dy, Eu, Tb, Nd, Yb, Er, Ho, Lu ;
∘ Ib : Cu, Ag, Au ;
∘ IIa : Ca, Mg ;
∘ IIIa : Ga, In ;
∘ IIIb : Y ;
∘ Va : Bi ;
∘ VIb : Cr, Mo ;
∘ VIIb : Mn, Tc ;
∘ VIII : Fe, Ru, Pt, Rh, Ir.
Gd, Dy conviennent bien e.g. pour des nanoparticules utiles comme agent de contraste en IRM

Eu, Tb, Nd, Yb, Er conviennent bien e.g. pour des nanoparticules utiles comme agent de fluorescence.

Ho, Lu conviennent bien e.g. pour des nanoparticules utiles comme agent de Curiethérapie. Lu, Yb, Gd, Ho conviennent bien e.g. pour des nanoparticules utiles comme agent de radio-sensibilisation.

Selon une caractéristique avantageuse de l'invention, les cations Mⁿ⁺ et/ou D^{m+} sont localisés à la surface des nanoparticules. D'où il s'ensuit que ces cations sont proches de molécules d'eau et peuvent ainsi avoir notamment un effet important de rehaussement de contraste T₁ en IRM. Cette amélioration des performances des nanoparticules utilisées suivant l'invention, est un témoin, parmi d'autres, de la localisation des cations Mⁿ⁺ et/ou D^{m+} à la surface.

### Nanoparticules ultrafines fonctionnalisées « sans coeur »

Dans un mode de réalisation plus particulièrement préféré, en raison notamment de leur très faible dimension, les nanoparticules utilisables selon l'invention sont obtenues à partir d'une nanoparticule précurseur comprenant:
- un cœur comprenant un oxyde et/ou un oxohydroxyde métallique (M) de terre rare, au moins en partie sous forme cationique Mⁿ⁺, n étant un nombre entier compris entre 2 et 4, éventuellement dopé par un dopant (D) présent au moins en partie sous forme cationique D^{m+}, m étant un nombre entier compris entre 2 et 6, de préférence un élément de transition ;
- au moins une couche d'enrobage comprenant des polyorganosiloxanes (POS);
- et, éventuellement un surenrobage comprenant un agent chélatant C1 apte à complexer les cations Mⁿ⁺ ou une molécule hydrophile apte à assurer la mise en suspension aqueuse des nanoparticules;
ladite nanoparticule précurseur étant soumise à une dissolution complète ou partielle du cœur M à l'aide d'un agent modificateur du pH et/ou d'un chélatant C2, identique ou différent à C1, apte à complexer tout ou partie des cations Mⁿ⁺ et D^{m+}, de sorte que le diamètre moyen de la nanoparticule ainsi obtenue est réduit à une valeur comprise entre 1 et 20 nm, de préférence entre 1 et 10 nm encore plus préférentiellement de 2 à 5 nm ou encore entre 1 et 5nm.

Ces nanoparticules obtenues selon le mode décrit ci-dessus ne comprennent pas de cœur encapsulé par au moins un enrobage. Il en résulte des tailles observées comprises entre 3 et 5 nm. On parle alors de nanoparticules ultrafines.

Ces nanoparticules dites « ultrafines » ou « sans cœur » sont éventuellement greffées à des molécules ciblantes, et en particulier des molécules ciblant les tissus des poumons comme décrites au paragraphe suivant.

De préférence, les agents chélatants C1 et/ou C2 sont présents dans les nanoparticules en quantité telle que tout ou partie des ions, en particulier les cations Mⁿ⁺ voire D^{m+} ne sont pas libres mais sont complexés. Mieux encore, le pourcentage massique des éléments M et D impliqués dans des liaisons M-O-M ou M-O-D ou D-O-D ou M-O-Si ou D-O-Si, par rapport au nombre total d'éléments M et D, inférieur ou égal à, dans un ordre croissant de préférence 10; 5; 2, 1; 0,5; 10⁻¹; 10⁻²; 10⁻³.

Le nombre de chélatants C1 et/ou C2 dans les nanoparticules est supérieur au nombre de cations restants Mⁿ⁺ et/ou de D^{m+}, voire d'autres cations (e.g. Ca⁺⁺, Mg⁺) éventuellement ajoutés en plus des Mⁿ⁺ et/ou de D^{m+}.

Ce pourcentage de liaisons (oxydes) M-O-M ou M-O-D ou D-O-D ou M-O-Si ou D-O-Si peut être mesuré par les techniques connues de type EXAFS, XPS, spectroscopie vibrationnelle, microscopie électronique en transmission couplée à des analyses structurales etc.

Cette mesure permet d'évaluer le nombre de liaisons spécifiques du cœur, cette mesure permet une mesure quantitative de la présence ou non du cœur, elle permet aussi d'évaluer les espèces M ou D qui pourraient facilement se dissoudre et se retrouver sous forme ionique en solution.

Les chélatants C1 peuvent être greffés en surface des particules de polysiloxane ou directement insérés au sein de la matrice POS. Une partie ou la totalité de ces chélatants sont destinés à complexer des cations Mⁿ⁺ (e.g. du gadolinium) voire D^{m+}. Une autre partie de ces chélatants peut servir à la complexation de cations endogènes pour assurer une bonne compatibilité avec les milieux biologiques rencontrés.

L'un des intérêts majeurs des nanoparticules ultrafines selon l'invention, est que M et /ou D puisse être un agent actif en imagerie (e.g. un agent de contraste) et/ou en thérapie.

En effet, M/Mⁿ⁺ (e.g. du gadolinium) voire D/D^{m+} présentent des propriétés remarquables pour des applications biologiques en imagerie et/ou en thérapie, comme par exemple des propriétés magnétiques, fluorescentes, radioactives (élément de numéro atomique élevé) ou radiosensibilisantes (X, gamma, béta, neutron).

Ces nanoparticules peuvent donc être utilisées comme agent de contraste dans des systèmes d'imagerie comme : l'IRM, la scintigraphie SPECT, la scintigraphie PET, l'imagerie par fluorescence, les scanners X.

Outre la fonctionnalisation chélatante, ces nanoparticules peuvent être modifiées (fonctionnalisation) en surface par des composés hydrophiles (PEG) et/ou chargées différemment pour adapter leur bio-distribution au sein de l'organisme et/ou permettre un bon marquage cellulaire, en particulier pour le suivi des thérapies cellulaires.

Elles peuvent être par exemple fonctionnalisées en surface par greffage de molécules ciblant les tissus pulmonaires, ou, du fait de leur passage dans le sang, par greffage de molécules ciblant certaines zones d'intérêt de l'organisme, en particulier de zones tumorales. De cette façon, on concentre l'agent porté par ces nanoparticules, dans la zone d'intérêt sans avoir à augmenter de manière très importante les quantités injectées comme c'est le cas actuellement.

La fonctionnalisation peut se faire également par des composés comportant un autre principe actif et/ou des composés luminescents (fluorescéine). Il en résulte des possibilités d'utilisations thérapeutiques comme agent radiosensibilisant en combinaison avec des radiothérapies, des neutronthérapies, comme agent radioactif pour des traitements de curiethérapie, comme agent pour la PDT (photodynamic therapy) ou comme agent de vectorisation de molécules à effet thérapeutique.

Une autre caractéristique de ces nanoparticules ultrafines est le maintien du caractère rigide des objets et de la géométrie globale des particules après injection. Cette forte rigidité tridimensionnelle est assurée par la matrice polysiloxane, où la majorité des siliciums sont liés à 3 ou 4 autres atomes de silicium via un pont oxygène. La combinaison de cette rigidité avec leur petite taille permet d'augmenter la relaxivité de ces nanoparticules pour les fréquences intermédiaires (20 à 60 MHz) par rapport aux composés commerciaux (complexes à base de Gd-DOTA par exemple), mais aussi pour des fréquences supérieures à 100 MHz présentes dans les IRM haut champ de nouvelle génération.

Cette rigidité, non présente dans les polymères, est aussi un atout pour la vectorisation et l'accessibilité des molécules ciblantes.

Par ailleurs, il doit être souligné que la biocompatibilité de ces nanoparticules n'est pas la moindre de leurs qualités.

De préférence, les nanoparticules selon l'invention, et en particulier selon le présent mode de réalisation, ont une relaxivité r₁ par ion Mⁿ⁺ est supérieure à 5 mM⁻¹(d'ion Mⁿ⁺).s⁻¹ préférentiellement 10 mM⁻¹(d'ion Mⁿ⁺).s⁻¹ pour une fréquence de 20 MHz. Par exemple, elles ont une relaxivité r₁ par nanoparticule compris entre 50 et 5000 mM-1.s-1. Mieux encore, ces nanoparticules ont une relaxivité r₁ par ion Mⁿ⁺ à 60 MHz qui est supérieure ou égale à la relaxivité r₁ par ion Mⁿ⁺ à 20 MHz. La relaxivité r₁ considérée ici est une relaxivité par ion Mⁿ⁺ (par exemple gadolinium). r₁ est extrait de la formule suivante : 1/T₁ = [1/T₁]ₑₐᵤ + r₁[Mⁿ⁺],

Dans une autre variante, les nanoparticules ont un rapport massique en agent de contraste IRM T1 de type lanthanide, de préférence en Gadolinium supérieur à 5%, par exemple compris entre 5% et 50%.

Plus de détails concernant ces nanoparticules ultrafines, leurs procédés de synthèse et leurs applications sont décrits dans la demande de brevet WO2011/135101, qui est incorporée par référence.

### Les molécules ciblantes

Les molécules ciblantes sont greffées en surface de la nanoparticule et/ou au sein de l'enrobage. On pourra utiliser un couplage classique avec des groupes réactifs présents, éventuellement précédé d'une étape d'activation. Les réactions de couplage sont connues de l'homme du métier et seront choisies en fonction de la structure de la couche superficielle de la nanoparticule et des groupements fonctionnels de la molécule ciblante. Voir par exemple, « Bioconjugate Techniques », G. T Hermanson, Academic Press, 1996*,* dans « Fluorescent and Luminescent Probes for Biological Activity », Second Edition, W. T. Mason, ed. Academic Press, 1999*.* Des méthodes de couplage préférées sont décrites plus loin. De préférence, ces molécules ciblantes sont greffées aux agents chélatants de nanoparticules selon la variante des nanoparticules ultrafines « sans cœur » telle que décrite au paragraphe précédent.

On choisira les molécules ciblantes en fonction de l'application envisagée.

Dans un mode de réalisation préféré, on choisira par exemple des molécules appropriées pour le ciblage actif des tumeurs, notamment des tumeurs du poumon. A titre d'exemple de molécules ciblantes pouvant être greffées sur les nanoparticules, on peut citer les molécules contenant le tripeptide RGD capable de reconnaitre l'intégrine αᵥβ₃. De tels peptides et leurs dérivés (notamment pentaptide cyclique) sont décrits notamment dans WO2004/026894.

Il a récemment été observé que l'intégrine αᵥβ₃ présentait un profil d'expression très particulier dans le cancer du poumon. Dans les tissus sains, cet hétérodimère est indétectable (à l'exception des ostéoblastes) alors qu'il est exprimé à environ 10⁵ copies par cellules essentiellement sur la face ventrale des cellules endothéliales des capillaires en néoformation ou en remodelage. L'intégrine est en effet engagée dans une liaison avec la matrice extracellulaire pour permettre l'ancrage et la mobilité des cellules. Son accessibilité par la voie sanguine est donc modérée. En ce qui concerne les cellules tumorales et en particulier du cancer du poumon, l'intégrine αᵥβ₃ est plus exprimée sur le front d'invasion de la tumeur et aussi par les cellules qui s'échappent de la tumeur pour former des métastases. Mais de plus, il a été constaté que la tumeur induit un remodelage des tissus normaux voisins, s'accompagnant d'une surexpression de l'intégrine αᵥβ₃ sur la surface des cellules normalement négatives. Ainsi, une administration de nanoparticules selon l'invention, par les voies aériennes, par exemple par nébulisation, lesdites nanoparticules comprenant en outre, greffée à leur surface, des molécules ciblant l'intégrine αᵥβ₃, tels que les peptides contenant le motif RGD, permet un ciblage particulièrement efficace des tumeurs du poumon, en particulier en comparaison avec une administration « classique » par la voie intraveineuse.

Il a en outre été constaté par les inventeurs que l'administration des nanoparticules par les voies aériennes permet d'atteindre la circulation sanguine, et donc d'autres tissus via le passage dans le sang des nanoparticules au niveau des poumons, et/ou leur absorption par le système cellulaire immunitaire. Ces nanoparticules peuvent ainsi réaliser un ciblage tumoral passif ou actif de tumeurs non pulmonaires, qui sont classiquement ciblées par des agents de contraste par injection intraveineuse.

Aussi, dans certains modes de réalisation, on choisira des molécules ciblant d'autres organes, et notamment les tissus cancéreux.

Des molécules ciblantes appropriées pour le ciblage des tissus tumoraux ont été décrites par exemple dans la publication internationale WO01/00621 et incluent, les dérivés d'ammonium quaternaires, aptamères, polypeptides, anticorps...

### Composés hydrophiles

Selon une variante de l'invention, les nanoparticules peuvent être fonctionnalisées à leur surface avec des composés hydrophiles choisis dans le groupe des polyols, de préférence dans le sous-groupe comprenant les glycols, les sucres et leurs mélanges; les dextranes, le PEG et le PPG étant particulièrement préférés.

Suivant une alternative, ces composés hydrophiles peuvent être choisis parmi ceux qui ont des masses molaires inférieures à 2.000 g/mol, de préférence inférieures à 800 g/mol. On donne ci-après des exemples de composés hydrophiles, avec leur masse molaire (Mw) préférée :
- Poly(éthylèneglycol)bis(carboxyméthyl)éther (PEG), 250< Mw<2000 g.mol⁻¹ ;
- Polyoxyéthylène bis(amine), 250< Mw<2000 g.mol⁻¹ ;
- O-Méthyl-O'-succinylpolyéthylèneglycol, Mw de l'ordre de 2000 g.mol⁻¹ ;
- Méthoxypolyéthylèneglycolamine Mw de l'ordre de 750 g.mol⁻¹ ;
- Acide succinique et mercaptosuccinique ;
- Sucres, en particulier le glucose et ses dérivés, par exemple les dextranes ;
- Acides aminés ou peptides hydrophiles (acide aspartique, acide glutamique, lysine, cystéine, sérine, thréonine, glycine...) ;
- Et leurs mélanges.

Plus généralement, les composés hydrophiles comprennent avantageusement des fonctions alcools ou acides carboxyliques ou amines ou amides ou esters ou éther-oxydes ou sulfonates ou phosphonates ou phosphinates et seront liées, de préférence de façon covalente, à au moins 10 % des atomes de silicium du POS de la matrice.

### Procédé d'obtention des nanoparticules selon l'invention

D'une façon générale, l'homme du métier pourra aisément fabriquer des nanoparticules utilisées selon l'invention. Plus précisément, on notera les éléments suivants :
Pour des nanoparticules de type cœur-coquille, à base d'une cœur d'oxyde ou d'oxohydroxyde de lanthanides, on pourra utiliser un procédé de fabrication utilisant un alcool comme solvant, comme décrit par exemple dans P. Perriat et al., J. Coll. Int. Sci, 2004, 273, 191 *;* O. Tillement et al., J. Am. Chem. Soc., 2007, 129, 5076 *et* P. Perriat et al., J. Phys. Chem. C, 2009, 113, 4038*.*

Pour la matrice POS, plusieurs techniques peuvent être employées, dérivées de celles initiées par Stoeber (Stoeber, W; J. Colloid Interf Sci 1968, 26, 62). On peut également utiliser le procédé employé pour l'enrobage comme décrit dans Louis et al (Louis et al., 2005, Chemistry of Materials, 17, 1673-1682) ou la demande internationale WO 2005/088314.

En pratique, pour la synthèse de nanoparticules ultrafines, on forme une nanoparticule de type cœur/coquille avec un cœur d'oxyde de lanthanide (par voie polyol modifiée) et une coquille de polysiloxane (par sol/gel), cet objet a par exemple une taille aux alentours de 10 nm (préférentiellement 5 nanomètres). Un cœur d'oxyde de lanthanide de taille très petite (adaptable inférieure à 10 nm) peut ainsi être élaboré dans un alcool par un des procédés décrits dans les publications suivantes : P. Perriat et al., J. Coll. Int. Sci, 2004, 273, 191 *;* O. Tillement et al., J. Am. Chem. Soc., 2007, 129, 5076 *et* P. Perriat et al., J. Phys. Chem. C, 2009, 113, 4038*.*

Ces cœurs peuvent être enrobés par une couche de polysiloxane en suivant par exemple un protocole décrit dans les publications suivantes : C. Louis et al., Chem. Mat., 2005, 17, 1673 *et* O. Tillement et al., J. Am. Chem. Soc., 2007, 129, 5076*.*

On greffe à la surface du polysiloxane des chélatants spécifiques des cations métalliques visés ; on peut également en insérer une partie à l'intérieur de la couche mais le contrôle de la formation du polysiloxane est complexe et le simple greffage extérieur donne, à ces très faibles tailles, une proportion de greffage suffisante.

On sépare les nanoparticules des résidus de synthèse par une méthode de dialyse ou de filtration tangentielle, sur une membrane comportant des pores de taille adaptée.

Le cœur est détruit par dissolution (par exemple en modifiant le pH ou en apportant des molécules complexantes dans la solution). Cette destruction du cœur permet alors un éparpillement de la couche de polysiloxane (selon un mécanisme d'effondrement ou de corrosion lente), ce qui permet d'obtenir en final un objet en polysiloxane de morphologie complexe dont les dimensions caractéristiques sont de l'ordre de grandeur de l'épaisseur de la couche de polysiloxane, c'est-à-dire beaucoup plus petit que les objets jusqu'à présent élaborés.

Le fait de retirer le cœur permet ainsi de passer d'une taille de particules d'environ 5 nanomètres de diamètre à une taille d'environ 3 nanomètres. De plus, cette opération permet d'augmenter le nombre de M (e.g. gadolinium) par nm³ en comparaison d'une nanoparticule de polysiloxane théorique de même taille mais comprenant du M (e.g. gadolinium) uniquement en surface. Le nombre de M pour une taille de nanoparticule est évaluable grâce au rapport atomique M/Si mesuré par EDX.

Sur ces nanoparticules, on peut greffer des molécules ciblantes, par exemple à l'aide de couplage par liaison peptidique sur un constituant organique de la nanoparticule, comme décrit dans Montalbetti, C.A.G.N, Falque B. Tetrahedron 2005, 61, 10827-10852*.*

On pourra également utiliser une méthode de couplage utilisant la « click chemistry » Jewett, J.C.; Bertozzi, C.R. Chem. Soc. Rev. 2010, 39, 1272-1279*,* et faisant intervenir des groupements du type :
-N₃, -CN, -C≡CH, ou l'un des groupements suivants :

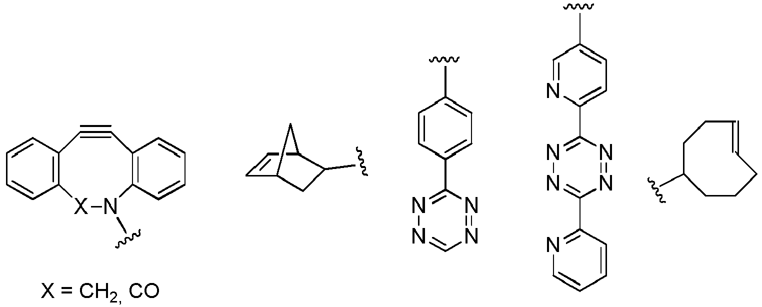

Dans un mode de réalisation spécifique, la nanoparticule selon l'invention comprend un chélatant présentant une fonction acide, par exemple le DOTA. On procède à l'activation de la fonction acide de la nanoparticule, par un exemple à l'aide d'EDC/NHS (1-éthyl-3-(3-diméthylaminopropyl)carbodiimide / *N*-hydrosuccinimide) en présence d'une quantité appropriée de molécules ciblantes. Les nanoparticules ainsi greffées sont ensuite purifiées, par exemple par filtration tangentielle.

### Mode d'administration des nanoparticules et aérosol contenant les nanoparticules

Selon une caractéristique essentielle de l'invention, les nanoparticules telles que définies ci-dessus, et en particulier, les nanoparticules ultrafines sans cœur, sont administrées par les voies aériennes, sous la forme d'aérosol.

Ainsi, l'invention porte sur un aérosol pour l'administration d'un agent de contraste ou thérapeutique chez l'homme ou l'animal par les voies aériennes, ledit aérosol comprenant, à titre d'agent de contraste ou thérapeutique, des nanoparticules telles que définies aux paragraphes précédents, et plus préférentiellement des nanoparticules ultrafines sans cœur, et, le cas échéant, un véhicule, fluide ou solvant pharmaceutiquement acceptable.

Par « voies aériennes », on entend l'ensemble des voies aériennes de l'appareil respiratoire, incluant la voie intranasale ou intratrachéale, en opposition notamment aux autres modes d'administration de médicament ou d'agent de contraste, constitués en particulier par la voie orale ou intraveineuse. L'objectif est d'atteindre les poumons par inhalation des nanoparticules.

Par « aérosol », on entend une suspension semi-stable de gouttelette liquides (contenant les nanoparticules) ou de particules solides dispersées dans un gaz de soutien, par exemple de l'air.

L'aérosol est ainsi obtenu à l'aide d'un dispositif approprié selon les paramètres axés sur la pharmacologie souhaitée ou sur le patient. Le dispositif permet par exemple l'atomisation d'un volume déterminé d'une formulation liquide contenant une suspension colloïdale de nanoparticules. Selon la formulation et la dose désirée, de tels dispositifs incluent, sans être limitatif, un aérosol-doseur, un inhalateur à poudre sèche, un inhalateur à base de liquide, un nébuliseur (à air comprimé/jet), un nébuliseur ultrasonique, un nébuliseur à membrane vibrante/à mailles). Pour une revue détaillée des inhalateurs utilisables (mais non limitative), voir en particulier M. B. Dalovich et al., Lancet, 2011, 377, 1032-1045*.*

Ainsi l'invention porte également sur une formulation des nanoparticules, comme définies dans les paragraphes précédents, notamment de nanoparticules ultrafines sans cœur, ladite formulation étant caractérisée en ce qu'elle est appropriée pour une administration par aérosolisation. La formulation peut être une formulation liquide contenant les nanoparticules sous la forme de suspension colloïdale stable, par exemple dans de l'eau stérile. Alternativement, il peut s'agir d'une matière sèche issue de la lyophilisation de ladite formulation liquide contenant la suspension colloïdale. Cette matière sèche est ensuite réhydratée dans un volume de liquide approprié avant utilisation.

L'invention porte également sur un inhalateur contenant une formulation liquide ou lyophilisée pour aérosolisation, comme décrit ci-dessus.

A titre d'exemple, pour une imagerie IRM des poumons, on préparera une formulation contenant une dose appropriée de nanoparticules ultrafines sans cœur, à base de gadolinium, et éventuellement un autre agent de contraste, lesdites nanoparticules étant en suspension dans l'eau. L'administration de cette formulation sous forme d'aérosol se fait à l'aide d'un nébuliseur sous la forme d'une solution saline, telle que décrite par exemple dans la partie expérimentale.

### Méthode d'imagerie médicale

L'invention se rapporte également à une méthode d'imagerie médicale non-invasive chez l'homme ou l'animal, comprenant les étapes suivantes :
(i) on administre des nanoparticules, telles que définies dans les paragraphes précédents, notamment des nanoparticules ultrafines sans cœur, à titre d'agent de contraste IRM T₁, par les voies aériennes, par exemple sous forme d'aérosol,
(ii) on capture les images IRM à l'aide d'une séquence IRM appropriée.

La méthode d'imagerie ne comporte ainsi aucune méthode invasive et est particulièrement confortable pour le patient. En particulier, elle ne comporte pas d'injection intraveineuse d'agent de contraste, celui-ci étant uniquement administré par les voies aériennes. Elle permet le cas échéant une diminution de la dose administrée, par une administration locale (dans les poumons) et une diminution des effets secondaires ou toxiques liées à l'administration des agents de contraste.

L'étape (i) a été décrite au paragraphe précédent.

Pour l'étape (ii), on procède classiquement à l'aide d'un appareil IRM, en choisissant la séquence IRM appropriée selon le patient, la zone d'intérêt à étudier et le type d'image souhaité, comme décrit par exemple dans « Comprendre l'IRM, manuel d'auto-apprentissage » de Bruno Kastler, Daniel Vetter. (Éditeur: Masson, 2011, ISBN: 9782294710445*).*

Les nanoparticules utilisées selon l'invention sont des agents de contraste multimodaux. Ainsi, on peut combiner avec une seule administration, plusieurs techniques d'imagerie et notamment l'IRM T₁, la scintigraphie PET (e.g ⁶⁴Cu) ou SPECT (¹¹¹In ou ^{99m}Tc), la fluorescence dans le proche infra-rouge, éventuellement, un agent radiosensibilisant, photosensibilisant ou approprié en radiothérapie, curiethérapie, neutronthérapie, photothérapie, etc...

Dans un mode particulièrement préféré, la méthode est appliquée à l'imagerie IRM des poumons, et en particulier, l'imagerie des tumeurs du poumon. Pour une imagerie des tumeurs des poumons, après administration de nanoparticules ultrafines sans cœur à base de gadolinium, on cherchera par exemple un contraste pondéré en T1, avec un temps d'écho court pour maximiser l'intensité du signal IRM pulmonaire. Les techniques d'IRM utilisées prendront en compte les spécificités de l'IRM pulmonaire, i.e. les mouvements respiratoires et cardiaques, la faible densité tissulaire et l'inhomogénéité magnétique de cet organe (« MRI of the Lung », Hans Ulrich Kauczor, Ed. Springer, 2009, ISBN 9783540346180*).*

Du fait de leur administration dans les poumons, une application évidente des méthodes d'imagerie selon l'invention concerne le diagnostic des pathologies pulmonaires, par exemple, les pathologies cancéreuses (tumeurs du poumon) et non-cancéreuses, et en particulier les pathologies pulmonaires inflammatoires, incluant par exemple l'asthme, la maladie pulmonaire obstructive chronique ou encore les broncho-pneumopathies chroniques obstructives.

L'invention a trait également à une méthode de suivi de l'efficacité thérapeutique d'un traitement thérapeutique chez l'homme ou l'animal, ladite méthode comprenant les étapes suivantes :
(i) à l'initiation du traitement, on administre au patient, des nanoparticules telles que définies ci-dessus, à titre d'agent de contraste, par les voies aérienne sous la forme d'aérosol,
(ii) on capture les images par une technique d'imagerie appropriée afin de visualiser les lésions,
(iii)on répète les étapes (i) et (ii) au cours du traitement du sujet, autant que nécessaire,
(iv)on déduit l'efficacité thérapeutique du traitement en comparant l'évolution des lésions au cours du traitement.

Une application particulière de cette méthode porte sur le suivi de l'efficacité thérapeutique d'un traitement chez l'homme ou l'animal, par exemple d'un traitement anti-tumoral, par exemple par chimiothérapie, radiothérapie, curiethérapie, photothérapie ou thermothérapie, contre les tumeurs des poumons.

Dans un mode de réalisation préféré, l'invention vise une méthode de suivi de l'efficacité thérapeutique d'un traitement anti-tumoral chez l'homme ou l'animal, notamment un traitement en chimiothérapie, radiothérapie, curiethérapie, photothérapie ou thermothérapie, dirigé contre les tumeurs des poumons, ladite méthode comprenant les étapes suivantes :
(i) on administre au patient atteint d'un cancer, à l'initiation du traitement, des nanoparticules, telles que définies dans les paragraphes précédents, notamment des nanoparticules ultrafines sans cœur, à titre d'agent de contraste, par les voies aériennes, par exemple sous forme d'aérosol,
(ii) on capture les images par une technique d'imagerie appropriée afin de détecter les tumeurs,
(iii)on répète les étapes (i) et (ii) au cours du traitement du patient avec un agent anti-tumoral,
(iv)on déduit l'efficacité thérapeutique de l'agent anti-tumoral en comparant les images des tumeurs obtenues au cours du traitement.

Ainsi, on peut suivre l'évolution des tumeurs, notamment de la taille des tumeurs au cours du temps, leur nombre et leur répartition, avant, pendant, et après le traitement du patient. On choisira naturellement des nanoparticules permettant un ciblage spécifique des tumeurs comme décrit plus haut.

Avantageusement dans les méthodes décrites ci-dessus, les nanoparticules peuvent être utilisées à la fois comme agent de contraste, du fait de leur propriété multimodale en imagerie, par exemple en tant qu'agent de contraste IRM T₁, et comme agent radio-sensibilisant, photosensibilisant ou radioactif pour le traitement des tumeurs.

Ainsi, dans la méthode décrite ci-dessus, dans un mode de réalisation particulier, les nanoparticules utilisées à titre d'agent de contraste sont les mêmes que celles utilisées à titre d'agent anti-tumoral.

Une autre application intéressante selon l'invention porte sur le suivi de l'efficacité thérapeutique d'affections pulmonaires, en particuliers des pathologies pulmonaires inflammatoires telles que l'asthme, la maladie pulmonaire obstructive chronique ou encore les broncho-pneumopathies chroniques obstructives, ou d'autres pathologies caractérisé par une augmentation de la part des vaisseaux dans la paroi bronchique (angiogenèse) et une hyperperméabilité des parois bronchiques.

### Applications thérapeutiques par aérosolisation des nanoparticules

En association avec un agent radio-sensibilisant pour la radiothérapie, ou radioactifs (et éventuellement la curiethérapie), pour la neutronthérapie, ou avec des agents pour la PDT (photodynamic therapy), les nanoparticules administrées dans les voies aériennes, sont particulièrement utiles pour le traitement des tumeurs, et notamment pour le traitement des tumeurs du poumon.

L'invention a donc également pour objet une composition pharmaceutique utilisable pour le traitement des tumeurs du poumon, par chimiothérapie, radiothérapie, curiethérapie, photothérapie ou thermothérapie, et comprenant des nanoparticules comme définies ci-dessus, par exemple, des nanoparticules ultrafines sans cœur, à titre d'agent radio-sensibilisant, photosensibilisant ou radioactif, éventuellement en combinaison avec un véhicule pharmaceutiquement acceptable et/ou d'autres principes actifs ou agents de contraste administrables par les voies aériennes.

### Méthode d'administration d'agent de contraste ou thérapeutique dans la circulation sanguine

Le mode d'administration par les voies aériennes permet une administration locale dans les poumons, mais également un passage dans la circulation systémique des nanoparticules. Ainsi, en couplant des molécules thérapeutiques sur les nanoparticules (couplage covalent ou non-covalent), l'invention propose l'utilisation de ces nanoparticules comme vecteur d'administration par les voies aériennes, dans la circulation sanguine, de molécules thérapeutiques ou d'agent de contraste. Selon le mode de couplage des molécules thérapeutiques, ces vecteurs peuvent être utilisées comme pro-drogue, à libération controlée, prolongée ou retardée du principe actif.

L'invention porte également sur une méthode de traitement d'un patient, comprenant l'administration par les voies aériennes sous la forme d'un aérosol d'une dose thérapeutiquement efficace de nanoparticules telles que définies dans les paragraphes précédents, notamment de nanoparticules ultrafines sans cœur, à titre d'agent thérapeutique.

### Légendes des figures

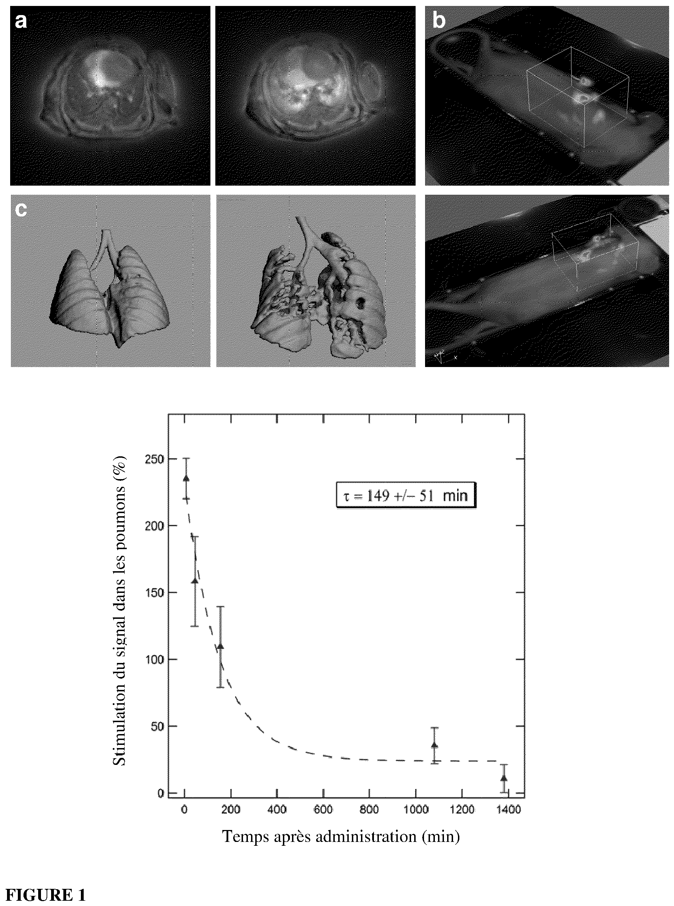

**Figure 1**: a) IRM des poumons chez la souris avant (gauche) et 15 minutes après (droite) l'administration intra-trachéale des nanoparticules SRP (40 microlitres avec une concentration de 20 mM en ion Gd³⁺). Un rehaussement de 183 % de l'intensité de l'image est observé après administration des nanoparticules, b) imagerie de fluorescence 3D de la souris, 1 heure après la nébulisation (50 µL) dans les poumons de nanoparticules SRP greffées avec la sonde fluorescente Cy5.5 , c) visualisation 3D des poumons avant (gauche) et après (droite) nébulisation de nanoparticules SRP (50 µL avec une concentration de 20 mM en ion Gd³⁺) obtenue en micro-tomographie X (les « trous » correspondent à la présence de nanoparticules), d) rehaussement du signal IRM dans les poumons en fonction du temps (instillation de 40 µL de solution de SRP avec une concentration de 20 mM en ion Gd³⁺), la constante de temps de détection des nanoparticules avant élimination par voie rénale est d'environ deux heures. Toutes les nanoparticules utilisées dans ces exemples ont été synthétisées suivant le protocole décrit dans l'exemple 1.

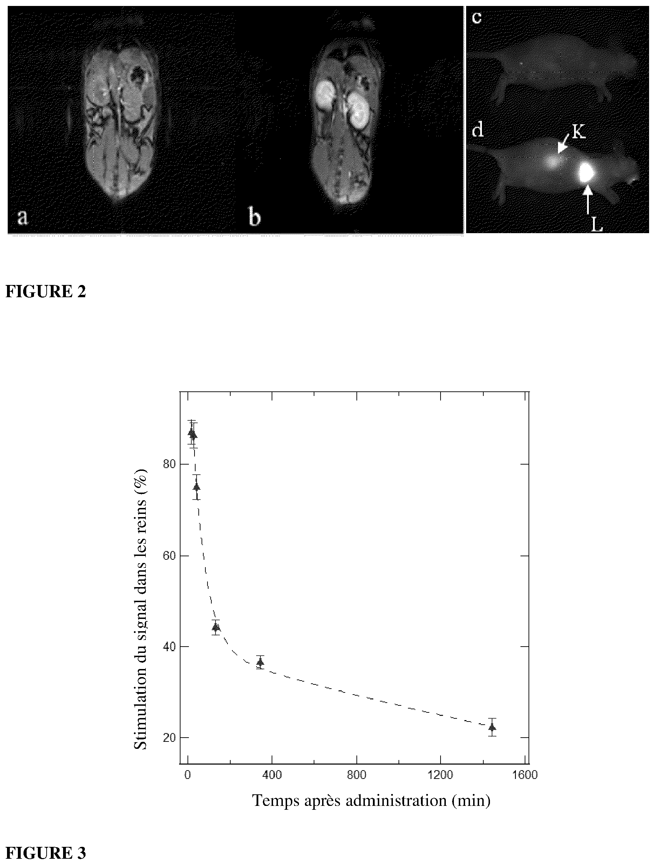

**Figure 2**: Images des poumons de souris obtenues avant (a) par IRM et c) par imagerie de fluorescence optique et 3 heures après l'administration intra-trachéale d'une solution de nanoparticules SRP obtenues suivant l'exemple 1 (couplées avec un fluorophore Cy 5.5 pour l'imagerie de fluorescence) (40 microlitres avec une concentration de 20 mM en ion Gd³⁺) (b) par IRM et d) par imagerie optique de fluorescence. Les images mettent en évidence l'élimination des nanoparticules par voie rénale (K (kidney) : rein ; L (lung) : poumon). Aucune élimination hépatique n'est détectée.
**Figure 3** : Evolution temporelle du rehaussement du signal IRM dans les reins suite à l'administration intra-trachéale d'une solution d'agent de contraste (50 mM Gd³⁺) obtenue suivant l'exemple 1. La présence de nanoparticules dans les reins est encore détectable 24 heures après l'administration de la solution.

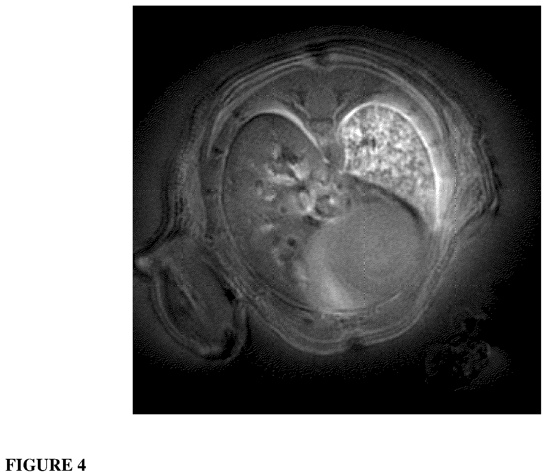

**Figure 4** : Image IRM haute résolution de la biodistribution de l'agent de contraste dans les poumons d'une souris après administration sélective de la solution (50 mM Gd³⁺) dans le poumon gauche (à droite dans l'image). Les nanoparticules utilisées ont été obtenues suivant l'exemple 1. Les principaux paramètres d'acquisition sont temps d'écho de 276 µs, épaisseur de coupe 0,5 mm, champ de vue de 2,5 cm, temps d'acquisition totale de 4 minutes.

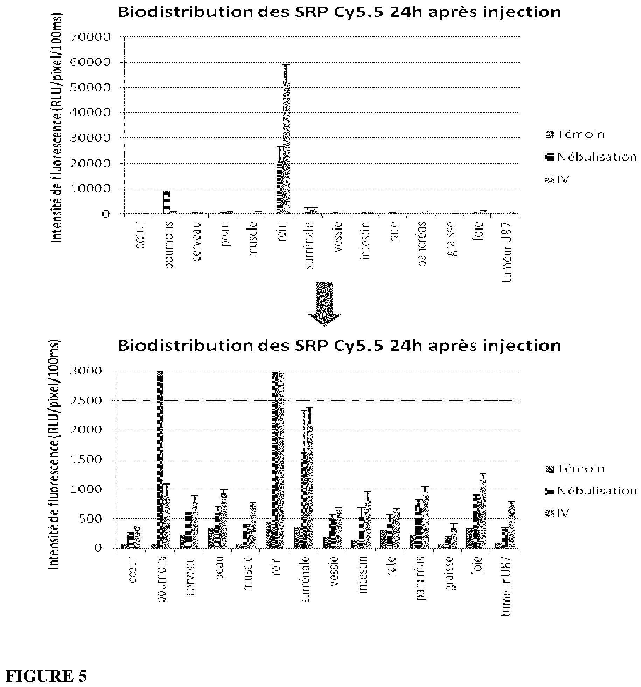

**Figure 5**: Biodistribution des SRP Cy5.5 24h après nébulisation intra-pulmonaire ou injection intra-veineuse d'une suspension de particules à 10mM en ions Gd³⁺. Les nanoparticules sont obtenues suivant l'exemple 1 avec couplage d'un fluorophore de type Cy 5.5 à la nanoparticule.

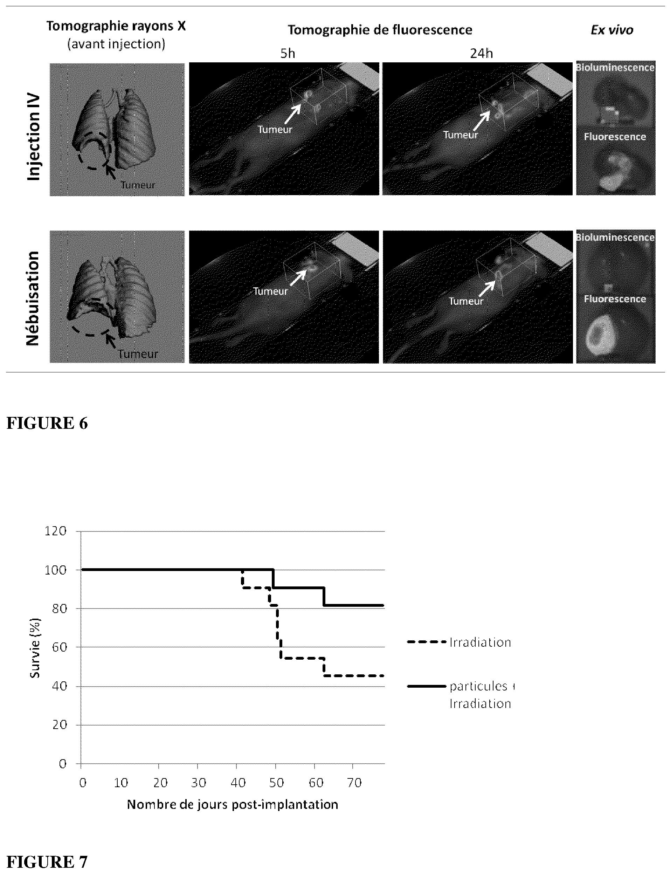

**Figure 6** : Ciblage passif de tumeurs pulmonaires H358-Luc orthotopiques après injection intra-veineuse ou nébulisation intra-pulmonaire d'une suspension de nanoparticules. Les nanoparticules sont obtenues suivant l'exemple 1 avec couplage d'un fluorophore de type Cy 5.5 à la nanoparticule.
**Figure 7** : Courbes de survie (Kaplan-Meier) obtenues sur des souris porteuses de tumeurs pulmonaires orthotopiques H358 traitées par irradiation seule 10 Gy ou traitées par irradiation 10 Gy réalisée 24h après administration intra-pulmonaire d'une suspension de nanoparticules à 20mM en ions Gd³⁺.

### Exemples

### Produits Chimiques

Le chlorure de gadolinium hexahydrate ([GdCl₃, 6H₂O], 99%), l'hydroxyde de sodium (NaOH, 99.99%), le tétraéthoxysilane (Si(OC₂H₅)₄, TEOS, 98%), l'aminopropyl triéthoxysilane (H₂N(CH₂)₃-Si(OC₂H₅)₃, APTES, 99%), la triéthylamine (TEA, 99.5%), l'hydrochlorate de N-(3-Diméthylaminopropyl)-N'-éthylcarbodiimide (EDC, >98.0%), le N-hydroxysuccinimide (NHS, >97.0%), le dianhydride diéthylènetriaminepentaacétique (DTPADA) et le diméthylsulfoxyde anhydre (DMSO, 99.5%) ont été obtenus auprès de Aldrich Chemicals (France). La Cy5.5 mono-NHS-ester a été commandée chez GE Healthcare. Le diéthylène glycol (DEG, 99%) provient de SDS Carlo Erba (France) tandis que l'acétone provient de Sodipro (France). Le 1,4,7,10-tétraazacyclododécane-1-glutarique anhydride-4,7,10-triacétique acide (DOTAGA) a été fourni par CheMatech SAS. Le cRGD tripeptide cyclique (Arg-Gly-Asp) provient de chez Genecust, Luxembourg. La 5-(4-carboxyphényl)-10,15,20-triphénylchlorine (TPC) provient de Frontier Scientific (Logan, Utah).

### Caractérisations

Les tailles moyennes des nanoparticules indiquées ont été mesurées par PCS (Photon corrélation spectroscopy) et correspondent à leur diamètre hydrodynamique. Ces mesures ont été prises sur un appareil de type Zetasizer NanoS (le laser servant à la PCS est un laser He-Ne 633 nm). Les mesures de zêtamétrie ont été réalisées sur le même appareillage en diluant préalablement les particules dans une solution 0.01M en NaCl. Le pH est ajusté à postériori. La microscopie à transmission électronique (TEM) est réalisée afin d'obtenir des données structurales et morphologiques sur les échantillons. Elle s'est déroulée en utilisant un microscope de type JEOL 2010 opérant à 200 kV. Les échantillons sont préparés par dépôt sur une grille de carbone. La relaxométrie à 1.4T (60 MHz) est mesurée grâce à un appareillage de type Bruker Minispec MQ60. La spectrométrie de masse est réalisée grâce à un spectromètre de type LTQ (Thermo Fisher Scientific, San Jose, CA). Les analyses élémentaires ont été réalisées au centre d'analyses du CNRS à Solaize par ICP-MS et ont permis de déterminer les taux de C, N, Si et Gd avec une précision minimum de 0.3%. Les analyses de fluorescence ont été obtenues sur un spectrofluorimètre de type Varian Carry Eclipse. La lyophilisation des particules est effectuée sur un appareil de type Christ Alpha 1,2 lyophilizer.

### Animaux

Les animaux utilisés pour la preuve de concept en IRM sont des souris femelles de type Balb/c âgées de 6 semaines et pesant entre 20 et 22 grammes, ces souris ont été achetées à l'élevage Janvier (Le Genest, Saint-Isle, France). Les animaux utilisés pour la preuve de concept en imagerie de fluorescence et tomographie rayons X sont des souris femelles de type NMRI nude âgées de 6 semaines et pesant environ 25 grammes, ces souris ont été achetées à l'élevage Janvier (Le Genest, Saint-Isle, France). Avant l'expérience, les souris s'acclimatent à leur nouvel environnement dans une salle à la température contrôlée pendant 1 semaine. Les expérimentations animales sont réalisées en respectant les consignes de l'INSERM (Institut National de la Santé Et de la Recherche Médicale) concernant le bien être animal.

### Outils d'imagerie :

### IRM

Les images d'IRM ont été acquises sur un spectromètre Brucker Biospec 47/50 avec un champ à 4.7 T (Brucker, Ettlingen, Allemagne), utilisant une bobine émettrice/réceptrice d'un diamètre intérieur de 25 mm (Rapid Biomédical, Rimpar, Allemagne). Les souris sont placées sur le ventre sur un berceau en plastique et gardées anesthésiées par un masque à gaz délivrant 2% d'isofluorane contenu dans un mélange de gaz N₂/O₂ (80 :20). La température du corps est maintenue constante grâce à une circulation d'eau chaude et le cycle respiratoire est surveillé constamment.

Pour chaque animal, 6 coupes axiales de 1 mm d'épaisseur sont acquises. L'acquisition est réalisée en respiration libre sans synchronisation respiratoire ou cardiaque à l'aide d'une séquence de type UTE (ultra short echo time) 2D multi-coupes. Les principaux paramètres sont temps d'écho 276 µs, temps de répétition de 84 ms, angle de bascule de 60°, champ de vue de 3 cm, temps d'acquisition total de 1 minute.

### Imagerie de fluorescence

Pour réaliser l'imagerie de fluorescence 2D ou 3D *in vivo,* les souris sont anesthésiées (isoflurane/oxygène : 3,5-4% pour l'induction et 1,5-2% pour le maintien).

Le système d'imagerie en 2D est composé d'un système d'excitation composé de diodes émettant à une longueur d'onde de 660 nm. Les images de fluorescence ainsi que les images « noir et blanc » sont réalisées avec une caméra CCD refroidie à -80°C (ORCAII-BT-512G, Hamamatsu, Massy, France), équipée avec filtre passe-haut RG 9 (Schott, Jena, Allemagne). L'acquisition des images ainsi que l'analyse est réalisée avec le logiciel Wasabi (Hamamatsu, Massy, France). 24h après l'injection les souris sont sacrifiées et les organes sont imagés. Une semi-quantification de la fluorescence des organes est obtenue en dessinant des régions d'intérêt autour des organes (ROIs).

La tomographie de fluorescence est réalisée à l'aide d'un système d'imagerie 3D fDOT. Le système est composé d'une boite noie, d'une lumière d'excitation provenant d'un laser (690 nm, 26 mW, Powertechnology, St Nom La Bretche, France) et d'une caméra CCD (ORCA ER, Hamamatsu) équipée d'un filtre passe-haut RG 9 (Schott, Jena, Allemagne). La cartographie 3D de la fluorescence dans la souris est calculée au moyen d'un algorithme de reconstruction.

### Imagerie de Rayons X

Pour réaliser l'imagerie rayons X et l'imagerie de fluorescence 2D ou 3D *in vivo,* les souris sont anesthésiées (isoflurane/oxygène : 3,5-4% pour l'induction et 1,5-2% pour le maintien).

Les images de tomographie rayons X sont réalisées avec le Scanco viva CT 40 (Scanco Médical, Inc., Bassersdorf, Suisse) en utilisant une énergie de 65 keV et un temps d'intégration de 200 ms. Les reconstructions 3D des poumons matérialisent la présence d'air dans les poumons. La présence des particules est donc représentée comme un « trou » sur les reconstructions, car les particules prennent en partie la place de l'air.

Nous possédons également un lit qui s'adapte dans le scanner X et le tomographe de fluorescence, permettant la superposition des images et la visualisation des colocalisations.

### Exemple 1 : Synthèse de Nanoparticules Hybrides de Gadolinium de type Gd-Si-DOTA : SRP (Small Rigid Platforms)

Les nanoparticules sont obtenues par une synthèse en 3 étapes telle que décrite dans WO2011/135101. Tout d'abord, les cœurs oxydes sont synthétisés dans le diéthylène glycol avant une croissance de la couche de polysiloxane suivie par le greffage covalent de complexants à la surface de la nanoparticule ; le cœur oxyde de gadolinium se dissout lors du passage dans l'eau ce qui entraine une fragmentation des particules permettant d'obtenir de petites particules de moins de 5 nanomètres composées uniquement de polysiloxane, d'espèces organiques et de complexants utiles en imagerie.

L'avantage de cette voie top down est de permettre l'obtention de très petites particules possédant des caractéristiques multimodales tout en présentant l'avantage d'être éliminables facilement par voie rénale.

### Description détaillée de la synthèse

### Cœurs oxydes

Une première solution est préparée en dissolvant 5,58 g de chlorure de gadolinium hexahydraté dans 500 mL de diéthylène glycol (DEG) anhydre à température ambiante. A cette première solution, on ajoute une deuxième solution de 500 mL de DEG contenant 4.95 ml de soude 10M. L'ajout se déroule à température ambiante pendant 24H. Une solution colloïdale transparente est obtenue avec une taille moyenne pour les cœurs oxydes de 3.5 nm de diamètre (mesures obtenues en PCS et en microscopie TEM).

### Encapsulation

La croissance de la couche de polysiloxane est assurée par une réaction de sol-gel en ajoutant deux précurseurs de silane (APTES et TEOS) en proportion 60/40 et de la triéthylamine comme catalyseur. Pour ce faire, 1050 µL d'APTES et 670 µL de TEOS sont ajoutés à la solution précédente sous agitation vigoureuse à 40°C. Dans le but d'obtenir une fluorescence dans le proche IR, une quantité variable d'APTES peut être couplée à de la Cy5.5 mono-NHS-ester par création de liaison amide entre l'APTES et le fluorophore. Après 1H de réaction, 2550 µL d'une solution de DEG contenant de la triéthylamine (0.1M de TEA, 10M d'eau) sont ajoutés. La réaction de sol gel précédemment décrite est répétée trois fois à chaque fois à 24H d'intervalle. A l'issue de ces différentes étapes, la solution est laissée sous agitation à 40°C pendant 48H. La solution colloïdale finale présente des particules d'une taille moyenne de 4.5 nm.

### Fonctionnalisation de surface

Finalement un large excès de DOTAGA (2 DOTAGA par atome de Gd) est ajouté aux particules permettant la formation d'une liaison peptidique entre les fonctions amines disponibles de la surface et une fonction acide carboxylique du ligand. 13,76 g de DOTAGA sont dispersés dans 200 mL de DMSO. Après l'ajout de la solution de particules à la solution contenant le DOTAGA, le mélange est laissé sous agitation pour 48 heures supplémentaires. Les nanoparticules sont ensuite précipitées dans 9L d'acétone. L'acétone est retirée et les particules sont lavées par 3L d'acétone supplémentaires et récupérées par centrifugation. Les particules sont ensuite redispersées dans 200 mL d'eau distillée, l'excès d'acétone est évaporé et ensuite la solution est maintenue sous agitation pendant 24H supplémentaires. La purification est assurée par centrifugation tangentielle à travers des membranes de type Vivaspin® (taille de pores : 5 kDa). Finalement, les solutions purifiées résultantes sont lyophilisées puis conservées au réfrigérateur pour plusieurs mois sans modification du produit. Pour obtenir des particules, plus fluorescentes, il est également possible d'ajouter la Cy5.5 en post-greffage c'est-à-dire après l'obtention des particules par réaction directe de la Cy5.5 mono-NHS ester avec les fonctions amines libres de la particule. L'analyse par fluorescence des particules a permis de démontrer qu'il est possible de greffer environ une Cy5.5 par particule. La taille de ces nanoparticules a été obtenue par spectroscopie de corrélation de fluorescence et a permis d'obtenir un diamètre hydrodynamique de 4 nm légèrement supérieur à celui obtenu sans fluorophore.

Les nanoparticules sans fluorophore ont un rayon hydrodynamique de 3±0.1 nm une fois rediluée en solution aqueuse. La masse des particules a été estimée à 8.5±1 kDa d'après une analyse en spectrométrie de masse. La relaxivité longitudinale r₁ est de 11.4 mmol⁻¹s⁻¹ à 60 MHz. A haut champ (300 MHz), la relaxivité obtenue à 300 MHz est de 6 mmol⁻¹s⁻¹. L'analyse élémentaire combinée à la taille des particules permet d'obtenir un total de 10 DOTA, 27 Si et 7 Gd par particules. Ces données ont été corroborées par un dosage potentiométrique et de fluorescence du nombre de DOTA libres présents par particule. Avant l'utilisation des particules pour les applications biologiques, les DOTA libres sont utilisés pour chélater d'autres ions Gd³⁺ afin de maximiser le r₁ par objet (114 mmol⁻¹.s⁻¹ par objet en moyenne) ou pour chélater des ions actifs en scintigraphie SPECT ou PET. Cette méthode est décrite dans la littérature (Lux et al., Ange. Chem. Int. Ed., 2011, 50, 12299). Une solution d'injection de ces nanoparticules est réalisée en diluant les particules dans une solution saline avec un tampon HEPES afin de fixer le pH. Une injection intraveineuse au niveau de la queue de rongeurs (rats ou souris) a permis de montrer une élimination rénale des particules combinée à une durée de vie plasmatique deux fois plus longue que le DOTAREM®.

### Exemple 2 : Fonctionnalisation des nanoparticules pour un ciblage actif des tumeurs : SRP-cRGD

Les nanoparticules utilisées pour le greffage sont similaires à celles décrites dans l'exemple 1. Elles possèdent donc un ensemble de DOTA libres (et ainsi de fonctions acides carboxyliques disponibles) pour le greffage du cRGD qui est connu pour être un cibleur de l'intégrine αᵥβ₃. Le greffage du cRGD à la particule est effectué grâce à un couplage peptidique entre une fonction acide carboxylique d'une unité DOTA et la fonction amine primaire du peptide cRGD. Les nanoparticules obtenues dans l'exemple 1 sont diluées dans l'eau (concentration voisine de 100 mM en Gd³⁺). Un mélange d'EDC et de NHS (3.4 EDC et 3.4 NHS par Gd) est ajouté aux particules, le pH est ajusté à 5 et le mélange est maintenu sous agitation pendant 30 minutes. Le cRGD (2.3 cRGD par Gd) est dissous à part dans du DMSO anhydre. Il est ensuite ajouté à la solution précédente et le pH du mélange est ajusté à 7.1 avant de le laisser sous agitation pendant 8 heures. La solution est finalement purifiée par filtration tangentielle à travers une membrane de 3 kDa avant d'être lyophilisée. L'analyse élémentaire permet de remonter à un taux de cRGD d'environ 2.5 par particule. Le même protocole est employé pour greffer le cRAD aux nanoparticules, ces nanoparticules serviront de contrôle lors des tests de ciblage actif réalisés avec le cRGD.

### Exemple 3 : Analyse de l'interaction de nanoparticules SRPcRGD obtenues suivant l'exemple 2 avec les cellules exprimant l'intégrine αvβ3 par cytométrie en flux et microscopie de fluorescence.

### Analyse par microscopie de fluorescence

Les cellules HEK293(β3), qui surexpriment l'intégrine αᵥβ₃, sont cultivées sur des lamelles dans des plaques 12 puits (100 000 cellules par puits) pendant une nuit à 37°C. Elles sont rincées une fois avec du PBS 1X, puis avec du PBS 1X contenant 1 mM de CaCl₂ et 1 mM de MgCl₂. Elles sont ensuite incubées pendant 30 minutes à 4°C (analyse de la fixation) ou 30 minutes à 37°C (analyse de l'internalisation), en présence de SRP (nanoparticules suivant l'exemple 1) avec un fluorophore de type Cy5.5, de SRP-cRGD (obtenues suivant l'exemple 2) Cy5.5 ou de SRP-cRAD (obtenues suivant l'exemple 2 et servant de témoin négatif) Cy5.5 à la concentration de 0,1 mM en ions Gd³⁺. Puis elles sont rincées avec du PBS Ca²⁺/Mg²⁺ (1 mM) et fixées (10 minutes avec paraformalhéhyde 0,5 %). Les noyaux sont marqués avec du Hoechst 33342 (5 µM pendant 10 minutes) (Sigma Aldrich, Saint Quentin Fallavier, France). Après un lavage avec du PBS 1X, les lamelles sont montées avec du Mowiol. Les images sont réalisées avec un microscope apotome (Carl Zeiss, Jens, Allemagne).

### Analyse par cytométrie en flux

Avant l'analyse de la fixation, les cellules adhérentes (HEK293(β3)) sont trypsinées, puis rincées une fois avec du PBS 1X froid (4°C), et une seconde fois avec du PBS 1X Ca²⁺/Mg²⁺ (1 mM) froid. Un million de cellules, dans un volume final de 200 µL est resuspendu dans une solution de SRP Cy5.5, de SRP-RGD Cy5.5 ou de SRP-RAD Cy5.5 à la concentration de 0,1 mM en ions Gd³⁺ et incubé pendant 30 minutes à 4°C. Après deux rinçages avec du PBS Ca²⁺/Mg²⁺ (1 mM), les cellules sont analysées rapidement par cytométrie en flux (LSR II, Becton Dickinson, France).

Pour l'analyse de l'internalisation, le protocole est similaire avec des réactifs à 37°C et une incubation de 30 minutes à 37°C.

Les résultats obtenus, en cytométrie en flux et en microscopie, ont permis de mettre en évidence une fixation et une internalisation spécifique des SRP-RGD Cy5.5 sur les cellules HEK293(β3), fixation qui n'est pas observée avec les SRP Cy5.5 et les SRP-RAD Cy5.5 (résultats non présentés).

### Exemple 4 : Imagerie IRM du poumon à l'aide de nanoparticules SRP synthétisées selon l'exemple 1

Une étude en concentration a été réalisée afin de déterminer la concentration d'injection la plus adaptée pour observer le meilleur contraste en IRM. Les souris ont été anesthésiées grâce à une injection intra péritonéale au moyen de 50 µg/g de kétamine (Panpharma, France) et de 5 µg/g de xylazine (Sigma-Aldrich, Saint-Quentin Fallavier, France). Après l'acquisition des images sans agent de contraste, les souris ont été intubées par voie intratrachéale au moyen d'un cathéter intraveineux 22-Gauge Teflon. Un volume de 50 µL de la solution de SRP a été introduit dans les poumons par le cathéter. 7 concentrations différentes en Gd³⁺ ont été testées (2, 5, 10, 20, 33, 50 et 100 mM) tandis qu'une solution saline a été injectée à une souris contrôle. Après l'arrêt de l'intubation, l'acquisition d'images des poumons a été réalisée à intervalles réguliers sur une période comprise entre 5 minutes et plusieurs heures après l'instillation de la solution. Parmi toutes ces concentrations, c'est la concentration de 50 mM qui a présenté le meilleur rehaussement de signal (235±15 %), le rehaussement de signal étant un peu moins important dans le cas de la concentration à 100 mM (171±10 %). Le rehaussement de signal est défini comme la différence entre le rapport signal à bruit dans les poumons avant et après l'administration, normalisée au rapport signal à bruit dans les poumons avant l'administration de l'agent de contraste.

La concentration de 50 mM a donc été retenue pour faire les études ultérieures de biodistribution par IRM. Une autre étude a été réalisée en utilisant cette concentration optimale de 50 mM sur 3 souris. Pour ces animaux, l'imagerie IRM des poumons, du foie, des reins et de la vessie a été réalisée à intervalles réguliers entre 5 minutes et 2 jours après l'administration de l'agent de contraste. Ces manipulations ont permis de démontrer que le temps de demi-vie de l'agent de contraste excrété par voie rénale est de 149±51 minutes. Cette élimination par voie rénale des nanoparticules est due à leur petite taille et représente un réel avantage en termes de toxicité de l'agent de contraste. Elle sera également importante pour une application originale décrite dans l'exemple 10.

### Exemple 5: Imagerie Rayons X et de fluorescence 2D et 3D du poumon à l'aide de nanoparticules SRP synthétisées suivant l'exemple 1.

Les SRP Cy5.5 lyophilisées obtenues suivant l'exemple 1 sont solubilisées dans un volume approprié d'eau pour obtenir une préparation injectable pendant au moins 30 minutes, afin d'obtenir une solution à 100mM en ions Gd³⁺. La suspension de particules est ensuite diluée dans une solution contenant un tampon HEPES et une solution saline. La préparation obtenue présente un pH de 7,4 et une osmolarité adaptée pour une administration chez l'animal.

La nébulisation intra-pulmonaire est réalisée à l'aide d'un microsprayer® (PennCentury®). Chaque souris est anesthésiée (mélange Domitor/Kétamine, injection intra-péritonéale) et reçoit 50 µL de suspension nanoparticulaire par voie intra-pulmonaire. Les souris utilisées pour la preuve de concept en imagerie de fluorescence sont des souris NMRI *nude* de 6 semaines pesant environ 25 grammes (Janvier, Le Genest Saint Isle, France).

Les résultats obtenus par imagerie rayon X et de fluorescence 2D et 3D ont montré une répartition des nanoparticules fluorescentes dans les deux poumons après la nébulisation. Ces résultats présentés à la figure 1 sont en parfaite adéquation avec ceux obtenus en IRM

### Exemple 6 : Synthèse de nanoparticules pour effet radiosensibilisant sur des tumeurs de poumons

Cet exemple décrit la synthèse de nanoparticules utilisées pour une utilisation en tant qu'agent de radiosensibilisation dans le cadre du traitement du glioblastome (G. Le Duc et al., ACS Nano, 2011, 5, 9566). Ces nanoparticules sont des nanoparticules d'oxyde de gadolinium entourées d'une couche de polysiloxane fonctionnalisée par du DTPA (complexe chélatant également un ion gadolinium). Elles sont envisagées pour un traitement en radiosensibilisation des tumeurs pulmonaires en raison de leurs caractéristiques très similaires aux nanoparticules décrites dans l'exemple 1 (tant du point de vue de la taille que de la morphologie).

La synthèse du cœur d'oxyde de gadolinium est effectuée en dissolvant le sel de chlorure de gadolinium hexahydraté (5.576 g) dans 100 mL de DEG à température ambiante et sous une agitation vigoureuse. La suspension est chauffée à 140°C jusqu'à la dissolution complète du sel (environ 1 heure). Lorsque la solution est devenue limpide, l'hydroxyde de sodium (4 mL, 3.38M) est ajouté goutte à goutte à la solution en maintenant une agitation vigoureuse. A l'issue de cet ajout, l'agitation et le chauffage sont maintenus pendant 3 heures. Une solution colloïdale transparente de cœurs d'oxyde de gadolinium est alors obtenue qui peut être conservée à température ambiante pendant plusieurs semaines sans altération.

La fonctionnalisation par la couche de polysiloxane s'effectue en présence des précurseurs silanes (APTES (10.1 mL) et TEOS (6.4 mL)) et d'une solution d'hydrolyse (triéthylamine dans le DEG (0.1 M en TEA et 10M en eau)). Ces solutions sont ajoutées en plusieurs étapes à 400 mL de la solution précédemment préparée contenant les cœurs oxyde de gadolinium ([Gd³⁺] = 45 mM) sous agitation à 40°C. Un taux 4 fois supérieur en silicium par rapport au gadolinium est choisi pour cette synthèse (le mélange des précurseurs est composé à 60% d'APTES et à 40% de TEOS). L'addition des différents précurseurs est réalisée en 6 étapes successives. Chaque étape consiste en l'ajout d'une partie de la solution contenant le mélange de précurseurs (5% de la solution pour la première étape, 15% pour la seconde et 20% pour les suivantes). Le délai entre chaque ajout est fixé à 1 heure. Après le dernier ajout, on laisse la solution sous agitation pendant 48 heures à 40°C. Afin de faciliter leur stabilité colloïdale en milieu biologique, les nanoparticules sont fonctionnalisées par le DTDTPA grâce à une liaison peptidique entre l'une des fonctions acide carboxylique activée et une fonction amine issue de l'APTES présente à la surface des nanoparticules. 100 mL de la solution colloïdale de nanoparticules précédemment obtenue sont ajoutés à 4.25g de DTDTPA dissous dans 20 mL de DMSO.

Les nanoparticules sont ensuite précipitées dans 500 mL d'acétone et le surnageant est enlevé par centrifugation. Puis la poudre blanche obtenue est lavée par un mélange éthanol/acétone (85/15), le surnageant est de nouveau enlevé par centrifugation (l'opération est répétée 3 fois). Les nanoparticules sont ensuite dispersées dans l'eau et purifiées par centrifugation tangentielle (à travers des membranes Vivaspin® de 5 kDa).

Après une purification par un facteur 1000, les particules sont lyophilisées. Elles sont ensuite directement injectables grâce à un tampon HEPES et une solution saline à la bonne osmolarité, le pH étant ajusté à 7.4. Ces nanoparticules présentent un diamètre hydrodynamique de 2 nm et un r₁ à 60 MHz de 9.4 mM⁻¹.s⁻¹ (le rapport r₁ sur r₂ est de 1.13). Elles peuvent donc être utilisées comme agents de contraste T₁ afin de visualiser leur biodistribution et leur accumulation dans la tumeur (G. Le Duc et al., ACS Nano, 2011, 5, 9566). Ce suivi en imagerie est important afin de déterminer le temps optimal auquel déclencher la radiothérapie. Ces agents thérapeutiques ont déjà prouvé leur pouvoir radiosensibilisant *in vitro* sur des cellules cancéreuses humaines du cerveau de type U87 (P. Mowat et al., Journal of Nanoscience and Nanotechnology, 2011, 11, 7833-7839*).* L'utilisation de ces nanoparticules ou bien de celles décrites dans les exemples 1 et 2 semble donc être particulièrement adaptée dans le cadre de la lutte contre le cancer du poumon en procédant à une thérapie (dans ce cas radiothérapie) guidée par imagerie.

### Exemple 7 : Protocole envisagé pour une application en radiosensibilisation de ces nanoparticules injectées par voie aérienne

Les nanoparticules synthétisées suivant l'exemple 1 sont injectées par voie aérienne. Leur accumulation dans la zone tumorale est ensuite repérée au moyen de l'IRM (exemple 4), de l'imagerie de fluorescence couplée à la tomographie X (exemple 5) ou encore de la scintigraphie après chélation d'un isotope radioactif utilisé en PET ou en SPECT. Une fois que la concentration en nanoparticules est repérée comme optimale dans la zone tumorale (rapport du contraste entre la zone saine et la zone tumorale) le traitement par radiothérapie peut être activé. Les nanoparticules sont ensuite éliminées par voie rénale après leur passage dans le sang. L'administration par voie aérienne permet d'injecter une moins grande quantité de nanoparticules au patient. Le traitement par radiothérapie étant réalisé de manière fractionnée en conditions cliniques, il sera alors possible de réaliser une nouvelle administration de particules avant chacune des séances en raison des possibilités d'élimination des particules et des quantités relativement faibles injectées. Les différents essais réalisés préalablement sur des tumeurs de type U87 radiorésistantes laissent prévoir des résultats intéressants en radiosensibilisation du poumon.

### Exemple 8 : Théranostic pour l'asthme

Aucune technique d'imagerie non-invasive ne permet actuellement d'évaluer la sévérité et l'étendue du remodelage bronchique chez les asthmatiques sévères. Le remodelage bronchique correspond à une évolution anormale des tissus bronchiques et péri-bronchiques faisant suite à des inflammations bronchiques répétées. Ce remodelage se traduit par différentes évolutions histologiques : épaississement de la membrane sub-épithéliale, augmentation des dépôts de matrice extra-cellulaire, néo-angiogénèse, hypertrophie des glandes à mucus et accroissement de la masse des muscles bronchiques lisses. Le remodelage bronchique chez les asthmatiques sévères se traduit par un pronostic défavorable, une morbidité importante et une dégradation marquée de la fonction respiratoire, de plus les patients ne répondent pas aux thérapies habituelles. Les asthmatiques sévères représentent 10% de la population asthmatique soit 350 000 patients en France et plus de la moitié des coûts liés à cette pathologie. Pour le développement de traitements efficaces, il est crucial de disposer d'une technique d'imagerie non-invasive permettant d'évaluer précisément la sévérité du remodelage et la réponse aux traitements. L'utilisation d'aérosols de nanoparticules greffées avec des molécules ciblantes (par exemple le tripeptide cRGD pour l'intégrine αvβ3 tel que décrit dans l'exemple 2) devrait permettre l'imagerie de l'angiogenèse associée au remodelage bronchique pour le diagnostic et pour l'évaluation de l'efficacité thérapeutique du traitement de l'asthme sévère. Dans cet objectif, un modèle murin d'asthme chronique pourra être mise en place. Ces souris (femelles type Balb/c âgées de 6 semaines) recevront des injections intra-péritonéales d'ovalbumine (100 µg) ou de Dermatophagoides pteronyssinus (D.pter) (100 µg) pour sensibilisation puis des instillations intra-nasales des mêmes composés à intervalles réguliers (jours 14, 27, 28, 29, 47, 61, 73, 74 et 75) pour l'apparition d'un remodelage bronchique 15 semaines après le début de la sensibilisation.

### Exemple 9: Synthèse de nanoparticules pour effet de photothérapie dynamique sur des tumeurs de poumons

L'addition d'un photosensibilisateur sur les nanoparticules permet de leur donner un effet toxique sous l'action de la lumière. Néanmoins la lumière ne peut pas pénétrer dans le corps au delà de quelques centimètres et ce même avec les chromophores les plus adaptés (absorption dans la fenêtre de transparence des tissus, c'est-à-dire la zone du proche infrarouge). Le principal intérêt de l'approche par les voies aériennes est de pouvoir obtenir une concentration dans les tumeurs pulmonaires. Ces dernières peuvent ensuite être illuminées au moyen de la fibre optique d'un endoscope (évitant ainsi les problèmes de pénétration des tissus par la lumière).

La synthèse des nanoparticules ayant une action en PDT et pouvant être injectée par les voies aériennes est mise en place de la manière suivante :
Le chlorure de gadolinium hexahydraté (3.346 g) est placé dans 60 mL de DEG à température ambiante. La suspension est ensuite chauffée à 140°C sous une agitation vigoureuse afin d'assurer la dissolution totale du sel de gadolinium. 4 mL d'une solution de d'hydroxyde de sodium à 2.03 M est ensuite ajoutée au goutte à goutte en maintenant l'agitation vigoureuse. La solution est ensuite laissée sous agitation à 180°C pendant 3 heures. Une solution colloïdale de cœurs d'oxyde de gadolinium est alors obtenue qui peut être stockée pendant à température ambiante pendant plusieurs semaines sans risque de dégradation.

L'ajout d'un photosensibilisateur dérivé de la chlorine (TPC) peut être réalisé grâce à l'activation de la fonction acide carboxylique de la TPC par un mélange EDC/NHS pour obtenir la 5,10,15, tri-(p-tolyl)-20-(p-carboxylphenyl)chlorinsuccinidyl ester (TPC-NHS) suivant le protocole décrit par C. Frochot et al. (Bioorganic Chemistry, 2007, 35, 205-220). 20 mg de TPC-NHS sont couplés par liaison peptidique avec 12.3 µL d'APTES dans 4.2 mL de DMSO anhydre pendant 1 nuit.

Les précurseurs de silane (APTES (1.5 mL) et TEOS (1.0 mL)) ainsi que la solution d'hydrolyse (solution aqueuse de triéthylamine dans le DEG (0.015 M de TEA et 1.5 M d'eau)) sont ajoutés par étape au 60 mL de DEG contenant les particules sous agitation à 40°C. L'ajout total est réalisé en 6 étapes. Chaque étape consiste en l'ajout d'une partie de la solution de précurseurs à la solution colloïdale dans le DEG (5% pour la première étape, 15% pour la suivante et 20% pour les dernières). La solution contenant la TPC couplée à l'APTES est ajoutée durant la première étape en même temps que les autres précurseurs. Le temps entre chaque addition est d'une heure. Après la dernière addition, le mélange est maintenu sous agitation pendant 48 heures à 40°C.

Les nanoparticules sont ensuite fonctionnalisées par le DTDTPA au moyen d'un couplage peptidique entre les amines de l'APTES et la fonction acide carboxylique activée du complexant. 2.5 g de DTDTPA dans 12 mL de DMSO anhydre sont ajoutés à la solution précédente. Le mélange résultant est alors agité pendant 1 heure. Les nanoparticules sont ensuite précipitées dans 300 mL d'acétone et le surnageant est retiré par centrifugation. La poudre obtenue est lavée par 3 fois par un mélange éthanol/acétone (85/15). La poudre est finalement redispersée dans l'eau et purifiée par centrifugation tangentielle sur membrane de 5 kDa (Vivaspin®). Cette procédure est répétée à plusieurs reprises afin qu'un taux de purification d'au moins 100 soit atteint. La solution purifiée de colloïdes est ensuite lyophilisée.

### Exemple 10 : Ciblage passif d'une tumeur sous-cutanée après administration intra-pulmonaire de nanoparticules.

Les particules cyanine 5.5 lyophilisées sont solubilisées dans volume approprié d'eau pour préparation injectable pendant au moins 30 minutes, afin d'obtenir une solution à 100mM en ions Gd³⁺. La suspension de particules est ensuite diluée dans une solution contenant un tampon HEPES et une solution saline. La préparation obtenue présente un pH de 7,4 et une osmolarité adaptée pour une administration chez l'animal.

Les souris ont préalablement reçu une greffe sous-cutanée de cellules tumorales sur le flanc. Lorsque la tumeur atteint une taille de 5x5mm, les souris reçoivent une injection intra-pulmonaire de nanoparticules (50 µL par souris). La nébulisation est réalisée à l'aide d'un microsprayer® (Penncentury®).

Les résultats obtenus ont montré un passage des particules très rapide dans la circulation sanguine (15-20 minutes) à partir des poumons, associé à une élimination rénale.

A partir de 5h après la nébulisation, un signal est détectable dans la tumeur sous-cutanée. Le signal augmente jusqu'à 24h après l'administration.

La biodistribution est comparable à celle obtenue pour l'administration d'une quantité équivalente d'ions Gd³⁺ par voie intra-veineuse (injection dans la veine caudale), mais avec une accumulation tumorale passive légèrement moindre (Figure 5). Les nanoparticules injectées par voie aérienne présentent l'avantage d'assurer une moins grande captation rénale.

### Exemple 11: Ciblage passif d'une tumeur pulmonaire orthotopique après administration intra-veineuse ou intra-pulmonaire de nanoparticules

Les particules cyanine 5.5 lyophilisées sont solubilisées dans un volume approprié d'eau pour préparation injectable pendant au moins 30 minutes, afin d'obtenir une solution à 100mM en ions Gd³⁺. La suspension de particules est ensuite diluée dans une solution contenant un tampon HEPES et une solution saline. La préparation obtenue présente un pH de 7,4 et une osmolarité adaptée pour une administration chez l'animal.

Les souris ont préalablement reçu une greffe orthotopique de cellules tumorales pulmonaires (H358) dans le poumon. Les cellules tumorales expriment le gène de la luciférase de manière stable, ce qui permet de suivre la croissance tumorale par imagerie de bioluminescence *in vivo.* Lorsque la tumeur est bien établie et détectable en imagerie de bioluminescence *in vivo,* les souris reçoivent une injection intra-veineuse (200µl par souris) ou une injection intra-pulmonaire (50µl par souris) de nanoparticules. La nébulisation est réalisée à l'aide d'un microsprayer® (Penncentury®).

Les résultats obtenus montrent qu'après administration intra-veineuse, les particules sont distribuées dans l'ensemble de l'organisme de la souris et sont éliminées par voie rénale. A partir de 5h après l'administration, un signal est détectable par imagerie de fluorescence3D dans la tumeur pulmonaire H358 implantée de manière orthotopique dans le poumon. Le signal est stable jusqu'à 24h post-administration.

En ce qui concerne l'administration intra-pulmonaire, les résultats montrent qu'après administration, les particules passent très rapidement dans la circulation sanguine (15-20 minutes) à partir des poumons, et sont éliminées par voie rénale. A partir de 5h après la nébulisation, la majeure partie des particules sont éliminées du poumon et un signal est détectable dans la tumeur pulmonaire implantée de manière orthotopique dans le poumon. Le signal est stable jusqu'à 24h post-administration.

Les imageries de fluorescence *in vivo* montrent une bonne colocalisation de la fluorescence avec la présence de la tumeur (tomographie rayons X réalisée avant l'administration des particules, voir figure 6).

Les imageries de fluorescence et de bioluminescence réalisées *ex vivo* sur les poumons montrent une colocalisation du signal de bioluminescence (correspondant aux cellules tumorales) et de la fluorescence (correspondant aux particules - Cy5.5) (voir figure 6).

Les particules sont donc capables de s'accumuler de manière passive dans des tumeurs pulmonaires implantées de manière orthotopique et ce quelle que soit la voie d'administration (intra-veineuse ou intra-pulmonaire).

### Exemple 12: Effet radiosensibilisant des nanoparticules sur des tumeurs pulmonaires après administration intra-pulmonaire.

Les particules lyophilisées sont solubilisées dans volume approprié d'eau pour préparation injectable pendant au moins 30 minutes, afin d'obtenir une solution à 100mM en ions Gd³⁺. La suspension de particules est ensuite diluée dans une solution contenant un tampon HEPES et une solution saline. La préparation obtenue présente un pH de 7,4 et une osmolarité adaptée pour une administration chez l'animal.

Les souris ont préalablement reçu une greffe orthotopique de cellules tumorales pulmonaires (H358) dans le poumon. Les cellules tumorales expriment le gène de la luciférase de manière stable, ce qui permet de suivre la croissance tumorale par imagerie de bioluminescence *in vivo.* Lorsque la tumeur est bien établie et détectable en imagerie de bioluminescence *in vivo,* les souris reçoivent une injection intra-pulmonaire (50µl par souris) de nanoparticules. La nébulisation est réalisée à l'aide d'un microsprayer® (Penncentury®). Vingt-quatre post-administration, les souris sont irradiées (rayons X) en dose unique dans un irradiateur conventionnel.

Les résultats obtenus montrent que l'administration intra-pulmonaire des nanoparticules avant l'irradiation améliore la survie des souris, en comparaison à une irradiation unique (voir figure 7). Les particules présentent donc un effet radiosensibilisant pour les tumeurs pulmonaires.

### MODES DE REALISATION

Des modes de réalisation sont décrits ci-après :
**1.** Nanoparticules, pour leur utilisation comme agent thérapeutique ou comme agent de contraste pour l'imagerie médicale, lesdites nanoparticules étant caractérisées en ce que
   a. elles présentent un diamètre moyen compris entre 1 et 20 nm, de préférence entre 1 et 10 nm, et encore plus préféré entre 1 et 5 nm,
   b. elles comprennent au moins un agent de contraste pour l'imagerie médicale et/ou un agent radio-sensibilisant ou radioactif pour la radiothérapie, ou un agent pour la neutronthérapie, la photothérapie ou, la thermothérapie,
   c. elles sont administrées chez l'homme ou l'animal par les voies aériennes, notamment intranasale ou intratrachéale.
**2.** Nanoparticules selon le mode 1, caractérisées en ce qu'elles comprennent une matrice de polyorganosiloxane.
**3.** Nanoparticules selon le mode 1 ou 2, caractérisées en ce qu'elles comprennent au moins un agent de contraste pour l'imagerie IRM T₁, et au moins un autre agent de contraste approprié pour l'une des techniques d'imageries suivantes :
   (iv) la scintigraphie PET ou SPECT,
   (v) la fluorescence dans le proche infra-rouge,
   (vi) la tomodensitométrie aux rayons X.
**4.** Nanoparticules selon le mode 3, caractérisées en ce qu'elles ont une relaxivité r1 par particule comprise entre 50 et 5000mM-1.s-1 et/ou un rapport massique en Gd d'au moins 5%, par exemple entre 5 et 50%.
**5.** Nanoparticules selon l'une des modes 1 à 4, caractérisées en ce qu'il s'agit de nanoparticules hybrides comprenant :
   - une matrice de polyorganosiloxane (POS) incluant, à titre d'agent de contraste ou radio-sensibilisant, des cations de terre rare Mⁿ⁺, n étant un nombre entier compris entre 2 et 4, éventuellement en partie sous forme d'un oxyde et/ou d'un oxohydroxyde métallique, éventuellement associé à des cations dopants D^{m+}, m étant un nombre entier compris entre 2 et 6, D étant de préférence une terre rare ou un élément de transition ;
   - un agent chélatant greffé à la matrice POS par liaison covalente -Si-C-, en quantité suffisante pour pouvoir complexer tous les cations Mⁿ⁺ et, le cas échéant, D^{m+}; l'agent chélatant ainsi greffé étant de préférence en excès par rapport aux cations Mⁿ⁺ et, le cas échéant, D^{m+};
   - le cas échéant, une molécule ciblante, pour le ciblage des nanoparticules, greffée à la matrice POS ou à l'agent chélatant.
**6.** Nanoparticules selon le modes 5, caractérisées en ce que l'agent chélatant est choisi parmi les agents complexants des lanthanides, notamment l'acide diéthylène triamine penta acétique (DTPA), l'acide 1,4,7,10-tetraazacyclododecane-1,4,7,10-tetra acétique (DOTA), l'acide 1,4,7-triazacyclononane-1,4,7 triacétique (NOTA) ou leurs dérivés.
**7.** Nanoparticules selon l'un des modes 1 à 6, caractérisées en ce que lesdites nanoparticules comprennent chacune, un agent de contraste, radioactif ou radio-sensibilisant choisi parmi un lanthanide, un oxyde et/ou un oxohydroxyde de lanthanide, le lanthanide étant de préférence choisi parmi Dy, Lu, Gd, Ho, Eu, Tb, Nd, Er, Yb ou leurs mélanges.
**8.** Nanoparticules selon l'un des modes 1 à 7 caractérisées en ce que la matrice POS de chaque nanoparticule est obtenue à partir d'une nanoparticule précurseur comprenant:
   - un cœur comprenant un oxyde et/ou un oxohydroxyde métallique M de terre rare, au moins en partie sous forme cationique Mⁿ⁺, n étant un nombre entier compris entre 2 et 4, éventuellement dopé par un dopant D présent au moins en partie sous forme cationique D^{m+}, m étant un nombre entier compris entre 2 et 6, de préférence une terre rare ou un élément de transition ;
   - au moins une couche d'enrobage comprenant des polyorganosiloxanes (POS);
   - et, éventuellement un surenrobage comprenant un agent chélatant C1 apte à complexer les cations Mⁿ⁺ ou une molécule hydrophile apte à assurer la mise en suspension aqueuse des nanoparticules;
   ladite nanoparticule précurseur étant soumise à une dissolution du cœur M à l'aide d'un agent modificateur du pH et/ou d'un agent chélatant C2, identique ou différent à C1, apte à complexer tout ou partie des cations Mⁿ⁺ et D^{m+}, de sorte que le diamètre moyen de la nanoparticule ainsi obtenue est réduit à une valeur comprise entre 1 et 20 nm, de préférence entre 1 et 10 nm encore plus préférentiellement de 1 à 5 nm.
**9.** Nanoparticules selon l'un des modes 1 à 8, caractérisées en ce qu'elles ont piégé un isotope radioactif susceptible d'être utilisé en scintigraphie, et de préférence choisi dans le groupe constitué par des isotopes radioactifs de In, Tc, Ga, Zr, Y, Cu ou Lu, par exemple ¹¹¹In, ^{99m}Tc, ⁶⁸Ga, ⁶⁴Cu, ⁸⁹Zr, ⁹⁰Y ou ¹⁷⁷Lu.
**10.** Utilisation des nanoparticules selon l'un des modes 1 à 9, comme agent de contraste pour l'imagerie médicale, notamment l'imagerie des poumons, caractérisée en ce que les nanoparticules sont administrées au patient par les voies aériennes, par exemple intranasale ou intratrachéale.
**11.** Utilisation des nanoparticules selon le mode 10, comme agent de contraste T₁ pour l'imagerie par résonnance magnétique.
**12.** Utilisation des nanoparticules selon le mode 11, comme agent de contraste T₁ pour l'imagerie par résonnance magnétique des tumeurs des poumons.
**13.** Utilisation des nanoparticules selon le mode 10, pour le ciblage des tumeurs non-pulmonaires.
**14.** Utilisation des nanoparticules selon l'un des modes 10 à 13, comme agent de contraste multimodal approprié pour l'imagerie IRM T₁, et une ou plusieurs des autres techniques d'imagerie choisies parmi :
   i. la scintigraphie PET ou SPECT,
   ii. la fluorescence dans le proche infra-rouge,
   iii. la tomodensitométrie aux rayons X.
**15.** Méthode d'imagerie médicale non-invasive chez l'homme ou l'animal, comprenant les étapes suivantes :
   i. on administre une composition comprenant des nanoparticules selon l'un des modes 1 à 9, à titre d'agent de contraste IRM T₁, par les voies aériennes, par exemple sous forme d'aérosol,
   ii. on capture les images IRM à l'aide d'une séquence IRM appropriée.
**16.** Méthode de suivi de l'efficacité thérapeutique d'un traitement thérapeutique chez l'homme ou l'animal, ladite méthode comprenant les étapes suivantes :
   (i) à l'initiation du traitement, on administre au patient, des nanoparticules selon l'un des modes 1 à 9, à titre d'agent de contraste, par les voies aérienne sous la forme d'aérosol,
   (ii) on capture les images par une technique d'imagerie appropriée afin de visualiser les lésions,
   (iii)on répète les étapes (i) et (ii) au cours du traitement du sujet, autant que nécessaire,
   (iv)on déduit l'efficacité thérapeutique du traitement en comparant l'évolution des lésions au cours du traitement.
**17.** Méthode selon le mode 16, pour le suivi d'une affection pulmonaire, tel que l'asthme, ou le cancer du poumon.
**18.** Aérosol pour l'administration d'un agent de contraste ou thérapeutique chez l'homme ou l'animal par les voies aériennes, ledit aérosol comprenant, à titre d'agent de contraste ou thérapeutique, des nanoparticules telles que définies selon l'un des modes 1 à 9, et, le cas échéant, un véhicule, fluide ou solvant pharmaceutiquement acceptable.
**19.** Nanoparticules selon l'un des modes 1 à 9, pour leur utilisation :
   (i) comme agents radio-sensibilisants ou radioactifs pour la radiothérapie,
   (ii) comme agents pour la neutronthérapie,
   (iii) comme agents pour la photothérapie ; ou,
   (iv) comme agents pour la thermothérapie,
   pour le traitement des tumeurs du poumon.

## Revendications

1. Nanoparticules, pour leur utilisation comme agent thérapeutique pour le traitement d'une tumeur, en particulier comme moyen de ciblage passif de tumeurs non pulmonaires, lesdites nanoparticules étant **caractérisées en ce que**
a. elles présentent un diamètre hydrodynamique moyen compris entre 1 et 5 nm tel que mesuré par spectroscopie de corrélation de photon,
b. elles comprennent au moins un agent radio-sensibilisant pour la radiothérapie, la curiethérapie, la neutronthérapie, ou des agents pour PDT (photodynamic therapy),
c. elles sont administrées chez l'homme ou l'animal par les voies aériennes, notamment la voie intranasale ou intratrachéale,
et **en ce qu'**il s'agit de nanoparticules hybrides comprenant :
• une matrice de polyorganosiloxane (POS) incluant, à titre d'agent radio-sensibilisant, des cations de terre rare Mⁿ⁺, n étant un nombre entier compris entre 2 et 4, éventuellement associé à des cations dopants D^{m+}, m étant un nombre entier compris entre 2 et 6, D étant de préférence une terre rare ou un élément de transition ;
• un agent chélatant greffé à la matrice POS par liaison covalente -Si-C-, en quantité suffisante pour pouvoir complexer tous les cations Mⁿ⁺ et, le cas échéant, D^{m+}.

2. Nanoparticules pour leur utilisation selon la revendication 1, **caractérisées en ce que** l'agent chélatant est choisi parmi les agents complexants des lanthanides, notamment l'acide diéthylène triamine penta acétique (DTPA), l'acide 1,4,7,10-tetraazacyclododecane-1,4,7,10-tetra acétique (DOTA), l'acide 1,4,7-triazacyclononane-1,4,7 triacétique (NOTA) ou leurs dérivés.

3. Nanoparticules pour leur utilisation selon l'une des revendications 1 à 2, **caractérisées en ce que** lesdites nanoparticules comprennent chacune, un agent radio-sensibilisant choisi parmi les lanthanides, par exemple choisi parmi Dy, Lu, Gd, Ho, Eu, Tb, Nd, Er, Yb ou leurs mélanges.

4. Nanoparticules pour leur utilisation selon l'une quelconque des revendications 1 à 3 **caractérisées en ce que** la matrice POS de chaque nanoparticule est obtenue à partir d'une nanoparticule précurseur comprenant:
- un cœur comprenant un oxyde et/ou un oxohydroxyde métallique de terre rare, au moins en partie sous forme cationique Mⁿ⁺, n étant un nombre entier compris entre 2 et 4, éventuellement dopé par un dopant D présent au moins en partie sous forme cationique D^{m+}, m étant un nombre entier compris entre 2 et 6, de préférence une terre rare ou un élément de transition ;
- au moins une couche d'enrobage comprenant des polyorganosiloxanes (POS);
- et, un surenrobage comprenant un agent chélatant C1 apte à complexer les cations Mⁿ⁺;
ladite nanoparticule précurseur étant soumise à une dissolution du cœur à l'aide d'un agent modificateur du pH et/ou d'un agent chélatant C2, identique ou différent à C1, apte à complexer tout ou partie des cations Mⁿ⁺ et D^{m+}, de sorte que le diamètre hydrodynamique moyen de la nanoparticule ainsi obtenue et tel que mesuré par spectroscopie de corrélation de photon est réduit à une valeur comprise entre 1 et 5 nm.

5. Nanoparticules pour leur utilisation selon l'une quelconque des revendications 1 à 4, **caractérisées en ce que** les nanoparticules comprennent chacune un agent radiosensibilisant choisi parmi un lanthanide, un oxyde, et/ou un oxohydroxde de lanthanide, le lanthanide étant un gadolinium.

6. Nanoparticules pour leur utilisation selon la revendication 5, **caractérisées en ce qu'**elles ont un rapport massique en cation gadolinium compris entre 5% et 50%.

7. Nanoparticules pour leur utilisation selon l'une quelconque des revendications 1 à 6, **caractérisées en ce qu'**elles sont utilisées à la fois comme agent de contraste IRM T1 pour l'imagerie de la tumeur et comme agent radio-sensibilisant pour le traitement de la tumeur.

8. Utilisation des nanoparticules telles que définies à l'une des revendications 1 à 6, comme agent de contraste pour l'imagerie d'une tumeur, **caractérisée en ce que** les nanoparticules sont administrées au patient par la voie aérienne, par exemple intranasale ou intratrachéale.

9. Utilisation des nanoparticules selon la revendication 8, comme agent de contraste T₁ pour l'imagerie par résonnance magnétique.

10. Utilisation des nanoparticules selon l'une des revendications 8 à 9, comme agent de contraste multimodal approprié pour l'imagerie IRM T₁, et une ou plusieurs des autres techniques d'imagerie choisies parmi :
i. la scintigraphie PET ou SPECT,
ii. la fluorescence dans le proche infra-rouge,
iii. la tomodensitométrie aux rayons X.

11. Méthode d'imagerie médicale non-invasive chez l'homme ou l'animal de tumeurs, comprenant les étapes suivantes :
i. on administre une composition comprenant des nanoparticules telles que définies à l'une quelconque des revendications 1 à 6, à titre d'agent de contraste IRM T₁, par les voies aériennes, par exemple sous forme d'aérosol,
ii. on capture les images IRM à l'aide d'une séquence IRM appropriée.

12. Nanoparticules telles que définies selon l'une des revendications 1 à 6, pour leur utilisation dans une méthode de suivi de l'efficacité thérapeutique d'un traitement anti-tumoral chez l'homme ou l'animal, ladite méthode comprenant les étapes suivantes :
i. on administre à l'homme ou l'animal atteint d'un cancer, à l'initiation du traitement, des nanoparticules telles que définies à l'une quelconque des revendications 1 à 6, par les voies aériennes, par exemple sous forme d'aérosol,
ii. on capture les images par une technique d'imagerie appropriée afin de détecter les tumeurs,
iii. on répète les étapes (i) et (ii) au cours du traitement avec un agent anti-tumoral.
**iv.** on déduit l'efficacité thérapeutique de l'agent anti-tumoral en comparant les images des tumeurs obtenues au cours du traitement.

13. Inhalateur, contenant une formulation pour aérosolisation, ladite formulation contenant une suspension colloïdale stable de nanoparticules telles que définies à l'une quelconque des revendications 1 à 6 ou une matière sèche issue de la lyophilisation de ladite formulation liquide contenant la suspension de nanoparticules.

14. Aérosol pour l'administration d'un agent de contraste ou thérapeutique chez l'homme ou l'animal par les voies aériennes, ledit aérosol comprenant à titre d'agent de contraste ou thérapeutique, des nanoparticules telles que définies à l'une quelconque des revendications 1 à 6, et, le cas échéant, un véhicule, fluide ou solvant pharmaceutiquement acceptable.

15. Nanoparticules pour leur utilisation comme vecteur pour l'administration de molécules thérapeutiques dans la circulation sanguine d'un patient, lesdites molécules thérapeutiques étant couplées à des nanoparticules telles que définies à l'une quelconque des revendications 1 à 6, et **caractérisées en ce que** lesdites nanoparticules sont administrées par les voies aériennes.
